# EUROPEAN PATENT SPECIFICATION

(11) **EP 3 173 767 B1**
(45) Date of publication and mention of the grant of the patent: **11.05.2022**
(21) Application number: 16200216.6
(22) Date of filing: 23.11.2016
(51) Int. Cl.: G01N 15/14, G01N 15/10, G01N 15/04, G01N 35/00, G01N 33/493

(54) **SAMPLE TESTING SYSTEM AND INFORMATION PROCESSING METHOD**
PROBENPRÜFSYSTEM UND INFORMATIONSVERARBEITUNGSVERFAHREN
SYSTÈME DE TEST D'ÉCHANTILLON ET PROCÉDÉ DE TRAITEMENT DE L'INFORMATION

(30) Priority: 27.11.2015 JP 2015232373
(43) Date of publication of application: 31.05.2017
(73) Proprietor: SYSMEX CORPORATION, Kobe-shi Hyogo 651-0073 (JP)
(72) Inventor: Imai, Youichi, Hyogo, 651-0073 (JP); Kinishi, Motoi, Hyogo, 651-0073 (JP); Takano, Yuji, Hyogo, 651-0073 (JP); Koyama, Kazuaki, Fukuoka, 812-0011 (JP)
(74) Representative: Hoffmann Eitle

(56) References cited:
- EP-A2- 2 416 161
- US-A1- 2011 022 327

## Description

### FIELD OF THE INVENTION

The invention relates to a sample testing system, an information processing method.

### BACKGROUND

Japanese Patent Application Publication No. H07-49346 describes a urinary sediment testing apparatus that automatically classifies the particles in urine such as red blood cell and white blood cells, and a urine qualitative testing apparatus that tests chemical components in urine such as sugar and protein. The result of the urinary sediment test and the result of the urine qualitative test are displayed together on a display device.

Urine samples contain various types of particles in various states. Thus, there are some samples in which the particles cannot be classified accurately, and other some samples from which the urinary sediment testing apparatus obtains a classification result insufficient to determine the state of the sample. Such samples need to be closely observed by a laboratory technician in a visual test using a microscope.

A visual test requires observing the numbers and states of various particles and performing a detailed examination even on casts that hardly appear. Visual tests are therefore a large burden on laboratory technicians and are desired to be performed as efficiently as possible.

Document EP 2 416 161 A2, February 08, 2012 , relates to a urine sample testing apparatus that comprises a urine qualitative measuring section configured to acquire a measurement result for the each of urine qualitative measurement items; a urine sediment measuring section configured to acquire a measurement result for each of a plurality of urine sediment measurement items; an operation part that is operable by a user to specify a combination of one of the plurality of urine qualitative measurement items and one of the plurality of urine sediment measurement items; and information processing unit configured to determine whether or not a first measurement result of the urine sample obtained by the urine qualitative measuring section and a second measurement result of the urine sample obtained by the urine sediment measuring section have a predetermined relation with respect to the urine qualitative measurement item and the urine sediment measurement item included in the specified combination.

Document US 2011/022327 A1, January 27, 2011, relates to a urine work area manager that comprises a computing unit and a set of machine executable instructions stored in a memory for execution by the computing unit. The computing unit transfers commands and data between a urine test strip reader and the manager using a first interface, and transfers commands and data between a urine sediment analyzer and the manager using a second interface. In operation, the computing unit instructs the urine test strip reader to analyze a plurality of urine samples, receives a strip analysis result from the urine test strip reader, and generates a first set of instructions for controlling the urine sediment analyzer using the strip analysis result. The computing unit further instructs the urine sediment analyzer to analyze the plurality of urine samples with the first set of instructions and receives a sediment analysis result from the urine sediment analyzer using the second interface

### SUMMARY OF THE INVENTION

A first aspect of the invention relates to a sample testing system. The sample testing system according to this aspect includes: a sample analyzer that analyzes a particle in a sample; and an information processing apparatus that receives a result of an analysis on the sample received from the sample analyzer. The information processing apparatus comprises a controller, a display unit, and an input unit. The controller displays a screen on the display unit, the screen including a reference-information display region for the result of the analysis on the sample, received from the sample analyzer, and an input-value display region for a visual test to be performed on the sample with a microscope when retesting is determined as necessary, basing the result of the analysis on the sample. With the screen displayed, the controller receives a count value of the particle counted in the visual test on the sample, through the input unit, and displays the received count value in the input-value display region.

A second aspect of the invention relates to an information processing method of inputting a result of a visual test on a sample containing a particle. The information processing method according to this aspect includes: displaying a screen on a display unit, the screen including a reference-information display region for a result of an analysis obtained by a sample analyzer analyzing the particle in the sample, and an input-value display region for the visual test to be performed on the sample with a microscope when retesting is determined as necessary, basing the result of the analysis on the sample; and with the screen displayed, receiving a count value of the particle counted in the visual test on the sample, through an input unit, and displaying the received count value in the input-value display region.

According to the invention, a visual test can be performed efficiently.

### BRIEF DESCRIPTION OF DRAWINGS

Fig. 1 is a diagram illustrating a schematic configuration of a sample testing system according to an embodiment.
Fig. 2 is a block diagram illustrating the configuration of the sample testing system according to an embodiment.
Fig. 3 is a schematic diagram illustrating information transmitted between analyzers and an information processing apparatus according to an embodiment.
Figs. 4A to 4D are schematic diagrams illustrating the configurations of a patient information table, a sample information table, a measurement order table, and an analysis result table, respectively, in a database according to an embodiment.
Fig. 5 is a schematic diagram illustrating the configuration of a screen including a sample list display region according to an embodiment.
Fig. 6 is a schematic diagram illustrating a screen including an input-value display region and a reference-information display region including a patient-information display region, a sample-information display region, a report-comment display region, a review-comment display region, a research-information display region, and a rule-comment display region according to an embodiment.
Figs. 7A to 7C are schematic diagrams exemplarily illustrating windows for inputting the count values o f particles counted in a visual test according to an embodiment.
Fig. 8 is a schematic diagram illustrating the configuration of a reference-information display region displaying the result of a urine qualitative analysis, the result of a urinary sediment analysis, and the result ofa visual test next to each other according to an embodiment.
Fig. 9 is a schematic diagram illustrating a state where analysis items and test items are rearranged according to an embodiment.
Fig. 10 is a schematic diagram illustrating the configuration of a reference-information display region displaying the result of an analysis that is useful from a research point of view and the result of a visual test next to each other according to an embodiment.
Fig. 11 is a schematic diagram illustrating the configuration of a reference-information display region displaying graphs according to an embodiment.
Fig. 12 is a schematic diagram illustrating the configuration of a reference-information display region displaying analysis values and the results of visual tests in time-series order according to an embodiment.
Fig. 13 is a schematic diagram illustrating the configuration of a reference-information display region displaying graphs in time-series order according to an embodiment.
Fig. 14 is a schematic diagram illustrating the configuration of a screen including an input-value display region for a body-fluid sample and a reference-information display region displaying the result of an analysis on the body-fluid sample and the result of a visual test on the body-fluid sample next to each other according to an embodiment.
Figs. 15A and 15B are flowcharts illustrating processing performed by a controller of the information processing apparatus according to an embodiment.
Figs. 16A and 16B are flowcharts illustrating processing performed by the controller of the information processing apparatus according to an embodiment.
Figs. 17A and 17B are flowcharts illustrating processing performed by the controller of the information processing apparatus according to an embodiment.

### EMBODIMENTS

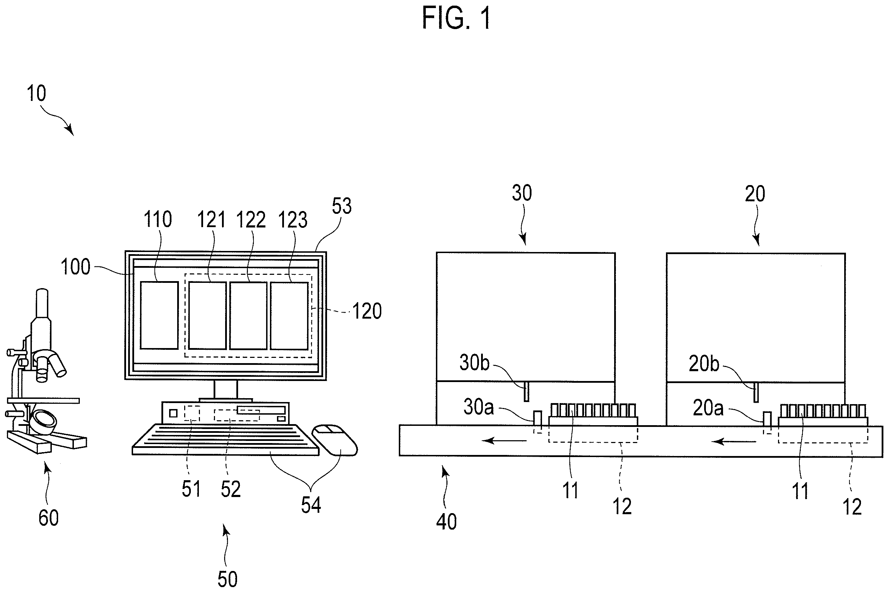

As illustrated in Fig. 1, sample testing system 10 includes urine qualitative analyzer 20, urinary sediment analyzer 30, transporting apparatus 40, information processing apparatus 50, and microscope 60. Transporting apparatus 40 transports sample racks 12 holding sample containers 11. Each sample container 11 contains a sample collected from a subject. The sample is collected from a patient since sample testing system 10 in this embodiment is assumed to be installed in a hospital.

Urine qualitative analyzer 20 analyzes chemical components in urine under urine qualitative analysis items. The analysis items refer to the types of chemical components to be analyzed, and the analysis items for urine qualitative analyzer 20 include glucose (GLU), protein (PRO), albumin (ALB), bilirubin (BIL), urobilinogen (URO), occult blood (OB), ketone body (KET), nitrite (NIT), white blood cell (WBC), and the like. The analysis items for urine qualitative analyzer 20 further include pH (PH), specific gravity (SG), color (COL), and the like.

When sample container 11 is positioned in front of barcode reader 20a, urine qualitative analyzer 20 reads barcode information from a label attached to this sample container 11 to acquire sample ID. According to acquired sample ID, urine qualitative analyzer 20 inquires of information processing apparatus 50 for a measurement order, and receives the measurement order from information processing apparatus 50. When sample container 11 is positioned directly below nozzle 20b, urine qualitative analyzer 20 aspirates the sample contained in sample container 11 through nozzle 20b. Urine qualitative analyzer 20 measures the sample according to the measurement order. Urine qualitative analyzer 20 performs an analysis, basing the result of the measurement, and transmits the result ofthe analysis to information processing apparatus 50.

If urine protein, urine sugar, urine occult blood, or the like is detected in the analysis by urine qualitative analyzer 20, urinary sediment analyzer 30 performs a test to check the presence and numbers ofurine particles, so as to estimate the type of kidney or urinary-system disease and its area.

Urinary sediment analyzer 30 analyzes urine particles under urinary sediment analysis items. The analysis items refer to the types of particles to be analyzed, and the analysis items for urinary sediment analyzer 30 include red blood cell (RBC), white blood cell (WBC), epithelial cell (EC), cast (CAST), and bacterium (BACT). The analysis items for urinary sediment analyzer 30 further include urine electric conductivity (Cond.). Note that urinary sediment analyzer 30 can also analyze research items such as crystal (XTAL), yeast-like fungi (YLC), small round cell (SRC), pathological cast (Path.CAST) including cell components or the like, mucus thread (MUCUS), sperm (SPERM), and red-blood-cell form information (RBC-Info.) for research purposes.

Urinary sediment analyzer 30 can further analyze components in body fluids. The "body fluids" in this case do not include blood, urine, or lymph but refer to body cavity fluids present in body cavities. Specifically, the body fluids refer to the spinal fluid, cerebrospinal fluid, pleural effusion, pleural fluid, pericardial fluid, joint fluid, synovial fluid, eye chamber fluid, aqueous humor, and the like. Dialysis fluid for peritoneal dialysis, peritoneal washing fluid, and the like are also included as some body fluids.

When sample container 11 is positioned in front of barcode reader 30a, urinary sediment analyzer 30 reads the barcode information from the label attached to this sample container 11 to acquire sample ID. According to acquired sample ID, urinary sediment analyzer 30 inquires of information processing apparatus 50 for a measurement order, and receives the measurement order from information processing apparatus 50. When sample container 11 is positioned directly below nozzle 30b, urinary sediment analyzer 30 aspirates the sample contained in sample container 11 through nozzle 30b. Urinary sediment analyzer 30 measures the sample according to the measurement order. Urinary sediment analyzer 30 performs an analysis, basing the result of the measurement, and transmits the result ofthe analysis to information processing apparatus 50.

A visual test using microscope 60 is performed when retesting is determined as necessary, basing the result of the analysis by urine qualitative analyzer 20 and the result of the analysis by urinary sediment analyzer 30. In this embodiment, as described later, information processing apparatus 50 determines whether or not a visual test needs to be performed, on the basis of the analysis results received from urine qualitative analyzer 20 and urinary sediment analyzer 30.

Information processing apparatus 50 receives and stores the results of the analyses by urine qualitative analyzer 20 and urinary sediment analyzer 30 and also displays the stored analysis results. Note that information processing apparatus 50 may not store the results of the analyses by urine qualitative analyzer 20 and urinary sediment analyzer 30 but only display the results of the analyses by urine qualitative analyzer 20 and urinary sediment analyzer 30. In this case, another apparatus that stores the results of the analyses by urine qualitative analyzer 20 and urinary sediment analyzer 30 is separately provided to sample testing system 10. Then, on the basis of the analysis results stored in this other apparatus, information processing apparatus 50 displays the analysis results and also receives input of the result of a visual test, as described later.

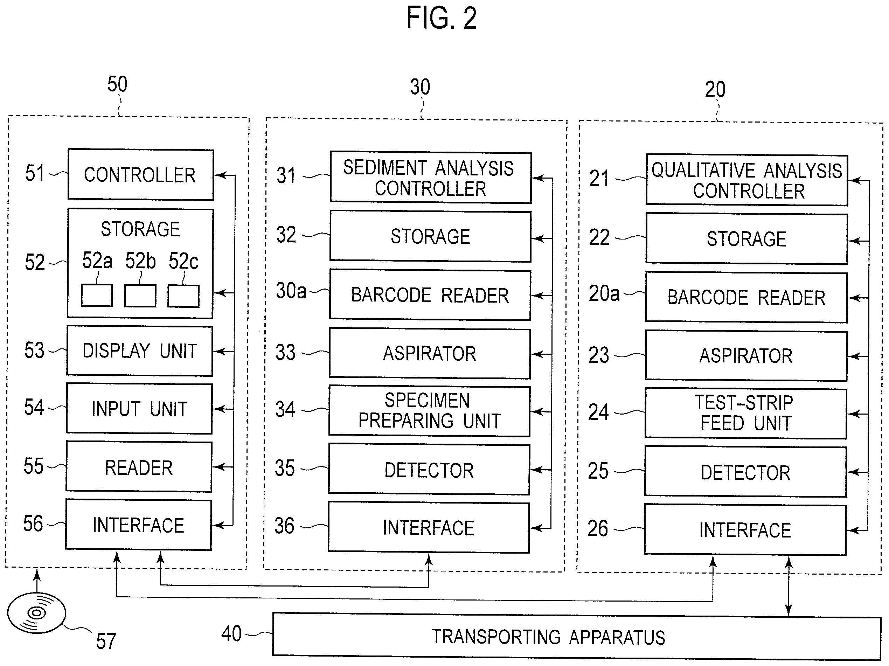

Information processing apparatus 50 includes controller 51, storage 52, display unit 53, and input unit 54. Controller 51 performs processing based on a program and data stored in storage 52. Storage 52 stores database 52b, as described later with reference to Fig. 2. Controller 51 stores the results of analyses on samples received from urine qualitative analyzer 20 and urinary sediment analyzer 30 into an analysis result table in database 52b, stored in storage 52. Controller 51 displays screen 100 including input-value display region 110 and reference-information display region 120 on display unit 53. Input-value display region 110 and reference-information display region 120 display information on a single selected sample.

Reference-information display region 120 is a region for displaying the results of analyses on a sample read from the analysis result table in database 52b, stored in storage 52. Input-value display region 110 is a region used for a visual test to be performed on a sample by using microscope 60. The operator performs a visual test by dropping, onto a microscope slide, a droplet of a sample for which a visual test has been determined to be necessary, and observing the sample on the microscope slide with microscope 60. Using input unit 54, the operator inputs the types of particles that he or she checked in the visual test. With screen 100 displayed, controller 51 receives the count values of the particles counted in the visual test on the sample, through input unit 54, and displays the received count values in input-value display region 110. Controller 51 stores the count values displayed in input-value display region 110 into the analysis result table in database 52b, stored in storage 52, as the result of the visual test.

Reference-information display region 120 includes regions 121 to 123. Region 121 is a region for displaying the result of an analysis on a sample by urine qualitative analyzer 20. Region 122 is a region for displaying the result of an analysis on a sample by urinary sediment analyzer 30. Region 123 is a region for displaying the result of a visual test on a target sample read from the analysis result table in database 52b, stored in storage 52.

With such display and processing, the operator can refer to the results of analyses on a sample on screen 100 as reference information when performing a visual test. Thus, the operator can figure out in advance what he or she should pay attention to and the like during the visual test, and then perform the visual test. This can make the visual test more efficient and accurate. Moreover, with screen 100 displayed, the operator can input the count values of the particles counted in the visual test, performed by using microscope 60, into information processing apparatus 50 through input unit 54. In this way, the operator can refer to the reference information and input the result of the visual test in a continuous manner. This can make the inputting of the visual test result more efficient.

Moreover, single screen 100 displays input-value display region 110 and region 123, in which to display the result of a visual test on a visual-test target sample read from the analysis result table in database 52b, stored in storage 52. In this way, the operator can refer to the result of a visual test already been performed by another operator as reference information. For example, in a case where two or more operators perform visual tests, an operator who is performing his or her visual test can refer to the visual tests already performed by the other operators as reference information. This can prevent mistakes and the like in a visual test and also make the visual test even more efficient and accurate.

Furthermore, regions 121 to 123 are displayed next to each other within reference-information display region 120. In this way, the operator can easily look over the analysis results in regions 121, 122 and the visual test result in region 123, which he or she refers to as reference information, within display unit 53. This can make a visual test more efficient.

As illustrated in Fig. 2, urine qualitative analyzer 20 includes barcode reader 20a, qualitative analysis controller 21, storage 22, aspirator 23, test-strip feed unit 24, detector 25, and interface 26. Qualitative analysis controller 21 is formed of a CPU, for example. Storage 22 is formed of a ROM, a RAM, and a hard disk drive, for example. Qualitative analysis controller 21 performs processing based on a program stored in storage 22. Qualitative analysis controller 21 controls parts of urine qualitative analyzer 20, controls transporting apparatus 40, and communicates with information processing apparatus 50 through interface 26.

Aspirator 23 aspirates the sample contained in sample container 11 through nozzle 20b, illustrated in Fig. 1, and transfers the aspirated sample to test-strip feed unit 24. Test-strip feed unit 24 takes out a test strip necessary for measurement from a test strip feeder, which stores test strips, and puts the sample onto measurement regions on the test strip thus taken out. Detector 25 captures images of the measurement regions on the test strip on which the sample has been put. Note that detector 25 may only need to measure reflected light, and may be a different reflected-light measurement system.

Qualitative analysis controller 21 performs an analysis based on each measurement result obtained by detector 25. Specifically, qualitative analysis controller 21 performs image processing on the captured image data to determine the state of color change at each of the measurement regions on which the sample has been put, and determines the analysis items corresponding to the measurement regions. The state of color change at each measurement region is divided into several levels. For example, the state of color change is divided into nine levels of "-", "±", "+", "2+", "3+", ..., "7+" in accordance with the concentration of the target component. Urine qualitative analyzer 20 thus performs nine-level semi-quantitative measurement as the urine qualitative measurement. Note that urine qualitative analyzer 20 may perform two-level qualitative measurement as the urine qualitative measurement. In this case, the state of color change at each measurement region is divided into two levels, such as "+" and "-", indicating the presence or absence of the component. Qualitative analysis controller 21 transmits the analysis results thus generated to information processing apparatus 50 on a sample-by-sample basis.

Urinary sediment analyzer 30 includes barcode reader 30a, sediment analysis controller 31, storage 32, aspirator 33, specimen preparing unit 34, detector 35, and interface 36. Sediment analysis controller 31 is formed of a CPU, for example. Storage 32 is formed of a ROM, a RAM, and a hard disk drive, for example. Sediment analysis controller 31 performs processing based on a program stored in storage 32. Sediment analysis controller 31 controls parts of urinary sediment analyzer 30 and communicates with information processing apparatus 50 through interface 36.

Aspirator 33 aspirates the sample contained in sample container 11 through nozzle 30b, illustrated in Fig. 1, and transfers the aspirated sample to specimen preparing unit 34. Specimen preparing unit 34 mixes the sample and a reagent to prepare a specimen for measurement. Detector 35 measures the specimen prepared by specimen preparing unit 34 with a flow cytometer.

Sediment analysis controller 31 performs an analysis based on the measurement result obtained by detector 35. Specifically, on the basis of detection signals obtained by detector 35, sediment analysis controller 31 classifies the particles in the measured specimen. Sediment analysis controller 31 counts each of the particles thus classified to obtain an analysis result for each analysis item. Sediment analysis controller 31 transmits the analysis results thus generated to information processing apparatus 50 on a sample-by-sample basis.

Note that, instead of performing the measurement using a flow cytometer, detector 35 may directly capture optical images of the forms of the particles in the sample. In this case, detector 35 is formed, for example, of a microscope with a function of automatically focusing on solid components' specimen images. Sediment analysis controller 31 processes the captured images to identify the components in the specimen.

Information processing apparatus 50 includes controller 51, storage 52, display unit 53, input unit 54, reader 55, and interface 56. Controller 51 is forme of a CPU, for example. Storage 52 is formed of a ROM, a RAM, and a hard disk drive, for example. Controller 51 performs processing based on various programs stored in storage 52. Controller 51 controls parts of information processing apparatus 50 and communicates with urine qualitative analyzer 20 and urinary sediment analyzer 30 through interface 56.

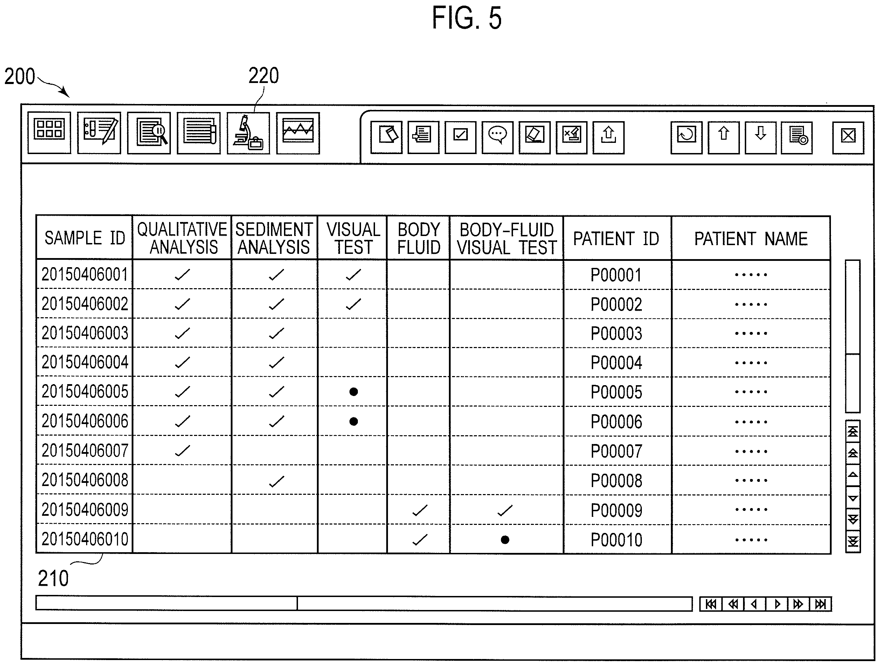

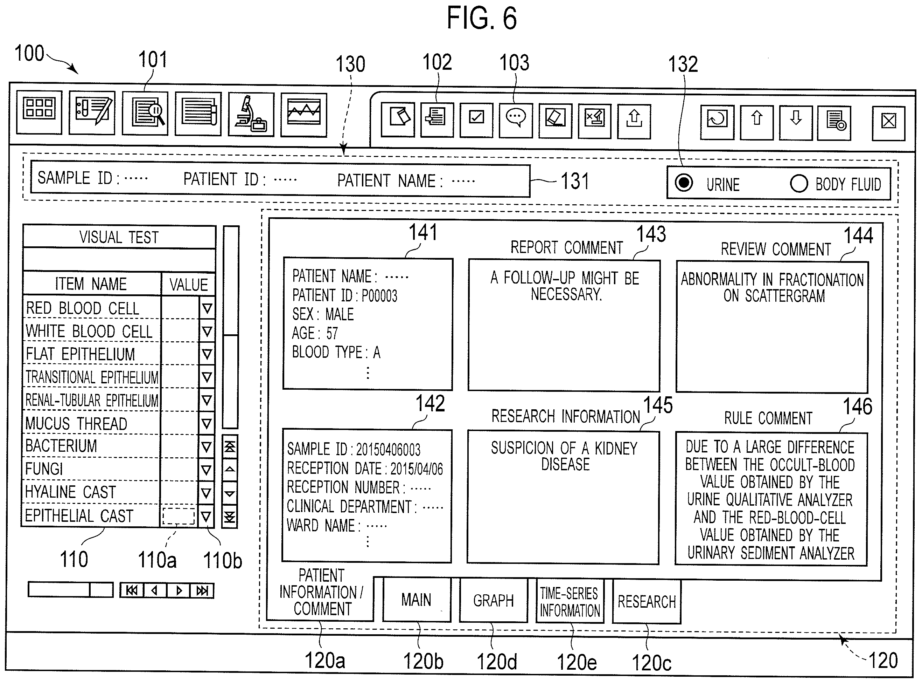

Storage 52 stores program 52a that causes the computer to perform processing for inputting the result of a visual test on a sample. Program 52a includes: a process of displaying screen 100 to be used for a visual test on display unit 53 and storing count values received through input unit 54 into the analysis result table in database 52b, stored in storage 52, as the result of the visual test; and the like. Program 52a also displays screen 200 for presenting a list of samples on display unit 53. Screen 200 is described later with reference to Fig. 5. Moreover, program 52a switches the information displayed in reference-information display region 120, in response to a display switch command. Detailed information displayed in reference-information display region 120 is described later with reference to Figs. 6 to 14. As described above, controller 51 executes program 52a to display a screen on display unit 53 and processes a command inputted through input unit 54.

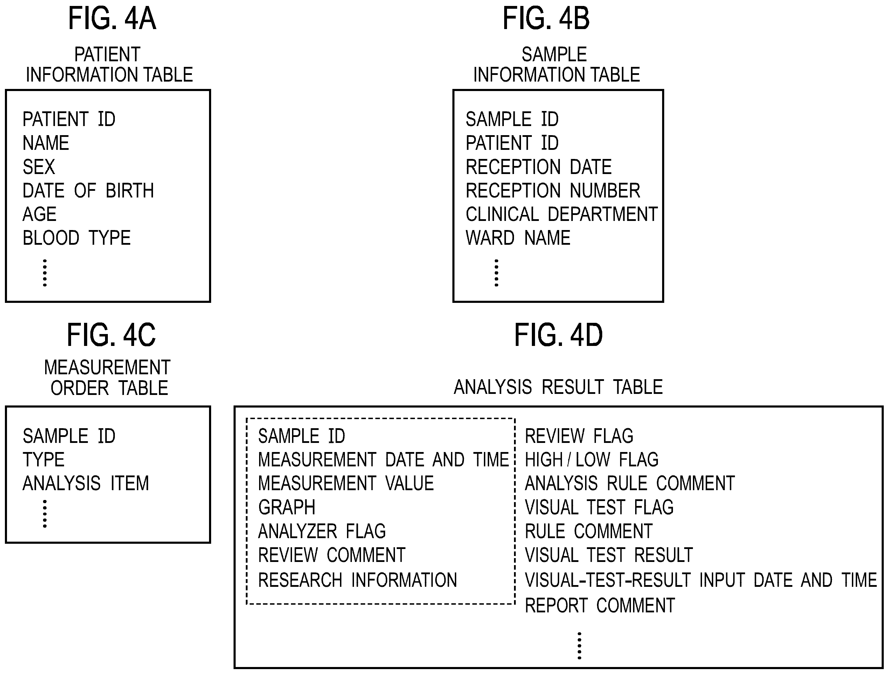

Storage 52 also stores database 52b for storing information on patients, information on samples, information on measurement orders, and analysis results. Database 52b is described later with reference to Figs. 4A to 4D. Storage 52 also stores rule 52c to be used to set a later-described review flag, high/low flag, analysis rule comment, visual test flag, and rule comment. Rule 52c includes pieces of rule information. With a setting screen displayed on display unit 53, the operator can change the contents of rule 52c through input unit 54. In this way, the operator can set appropriate rule 52c in accordance with the usage environment of sample testing system 10. Rule 52c is described later with reference to Fig. 4D.

Display unit 53 is formed of a display, for example. Input unit 54 is formed of a keyboard and a mouse, for example. Display unit 53 and input unit 54 may be formed integrally with each other, and may be formed of a touchscreen, for example. In this case, above-described screen 100, later-described screen 200, and the like are displayed on the touchscreen. By performing touch operations on the touchscreen, the operator can perform input operations similar to input operations using the keyboard and the mouse. Reader 55 is formed of an optical disk drive, for example. Reader 55 can read a program recorded in optical disk 57. In this way, program 52a, stored in storage 52, can be updated as needed.

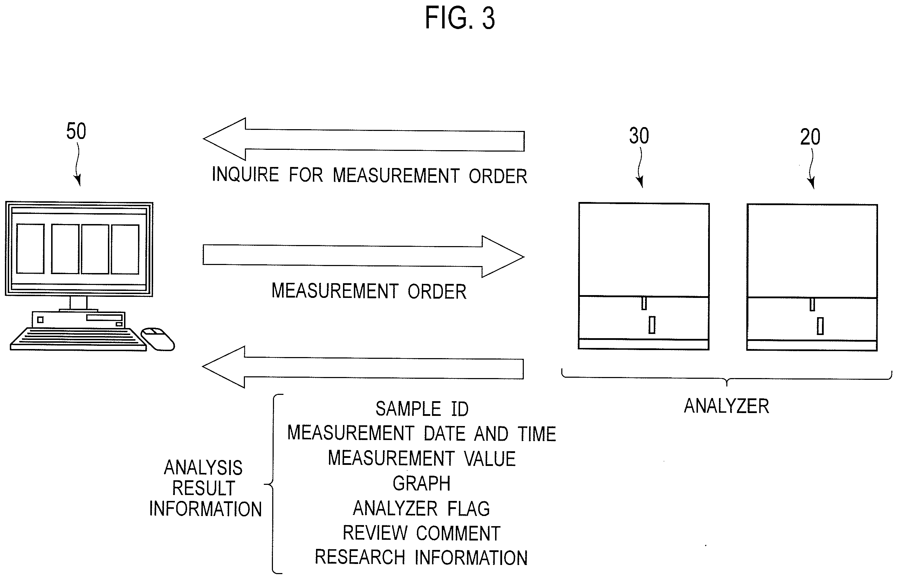

As illustrated in Fig. 3, urine qualitative analyzer 20 and urinary sediment analyzer 30 are together referred to as "analyzer" below. When the analyzer inquires of information processing apparatus 50 for a measurement order, information processing apparatus 50 transmits the measurement order to the analyzer. Urine qualitative analyzer 20 and urinary sediment analyzer 30 each performs measurement based on the measurement order. Qualitative analysis controller 21 and sediment analysis controller 31 each generates analysis result information based on the measurement result obtained by its analyzer. The analysis result information includes a sample ID, a measurement date and time, analysis values, graphs, analyzer flags, a review comment, research information, and the like. Urine qualitative analyzer 20 and urinary sediment analyzer 30 each transmits the analysis result information to information processing apparatus 50. Information processing apparatus 50 receives the analysis result information transmitted by the analyzer and stores the analysis result information in database 52b.

The pieces of information contained in the analysis result information are as follows. The sample ID is a number that allows unique identification of the sample. The measurement data and time are the data and time when the analyzer performed the measurement. Each analysis value is a value indicating an analysis result. The analysis value is, for example, a string of characters such as "Yellow", a numerical level such as "1+", or a numerical value such as "40.3", depending on the analysis item. The analysis result information includes an analysis value for each analysis item as the result ofthe analysis on the sample.

Each graph is a distribution chart indicating the distribution of a component(s) in the sample. For example, it is an image of a scattergram or histogram generated at the time of the analysis. The axes of the graph generated by urinary sediment analyzer 30 are set, basing the result of the measurement on the sample obtained by detector 35. The graph generated by urinary sediment analyzer 30 is, for example, an image of a scattergram in which the strengths of two different types of light resulting from particles in the sample are set on the vertical axis and the horizontal axis, respectively. Alternatively, it is, for example, an image of a histogram in which the strength of one type of light resulting from a particle in the sample is set on the horizontal axis, and its frequency is set on the vertical axis. Note that the graph is included only in the analysis result information generated by urinary sediment analyzer 30.

Each analyzer flag is a flag associated with an analysis value. The analysis result information includes an analyzer flag for each analysis item. The analyzer flag is, for example, a flag indicating that the analysis value is abnormal, a flag indicating that retesting is necessary, and a flag indicating that the reliability ofthe analysis value is low.

The review comment is annotative information on the analysis result. For example, urinary sediment analyzer 30 sets "abnormality in RBC-XTAL fractionation" as the review comment in a case of failing to fractionate red blood cells and crystals on a scattergram in the analysis. The research information is annotative information on the analysis result for research use. For example, urinary sediment analyzer 30 sets "suspicion of a kidney disease" as the research information in a case of obtaining an analysis result indicating the presence of many renal-tubular epithelial cells and an analysis result indicating unevenness in the form of the red blood cells. Thus, when a predetermined relation exists between the analysis values of at least two analysis items by either of urine qualitative analyzer 20 and urinary sediment analyzer 30, the analyzer sets, as the research information, annotative information on a disease judged from the predetermined relation.

As illustrated in Figs. 4A to 4D, database 52b includes a patient information table, a sample information table, a measurement order table, and the analysis result table.

As illustrated in Fig. 4A, the patient information table stores attribute information on patients. The patient information table includes items such as patient ID, name, sex, date of birth, age, blood type, past medical history, and patient health condition. As illustrated in Fig. 4B, the sample information table stores information on samples. The sample information table includes items such as sample ID, patient ID, reception date, reception number, clinical department, ward name, and private doctor name. As illustrated in Fig. 4C, the measurement order table stores information on measurement orders. The measurement order table includes items such as type and analysis item. The type indicates which one of urine or body fluid is the sample. The analysis item indicates each analysis item set for the target sample. The analyzer performs measurement for each analysis item and generates an analysis value for each analysis item from the measurement result.

As illustrated in Fig. 4D, the analysis result table stores information on analysis results. The analysis result table includes items such as sample ID, measurement date and time, analysis value, graph, analyzer flag, review comment, research information, review flag, high/low flag, analysis rule comment, visual test flag, rule comment, visual test result, visual-test-result input date and time, and report comment. In Fig. 4D, the items enclosed by the broken line, i.e. sample ID, measurement date and time, analysis value, graph, analyzer flag, review comment, and research information are stored according to the analysis result information received from the analyzer.

The review flag is a flag indicating whether retesting is necessary, determined for each analysis item by controller 51 with rule 52c. The high/low flag is a flag indicating whether the analysis value is high or low, determined for each analysis item by controller 51 with rule 52c. The analysis rule comment is annotative information generated on each analysis item by controller 51 with rule 52c. For example, when the analysis value of the analysis item for red blood cell is large, controller 51 sets annotative information "suspicion of hematuria" as the analysis rule comment for this analysis item.

The visual test flag is a flag indicating whether or not a visual test is necessary for the sample, determined by controller 51 with rule 52c. Controller 51 sets, as the visual test flag, a flag indicating that a visual test is necessary, when items in each table satisfy a condition included in rule 52c for determining that a visual test is necessary. For example, controller 51 sets, as the visual test flag, a flag indicating that a visual test is necessary, when there is a large difference between the analysis value of occult blood obtained by urine qualitative analyzer 20 and the analysis value of red blood cell obtained by urinary sediment analyzer 30.

The rule comment is annotative information on the sample generated by controller 51 with rule 52c. For example, when determining with rule 52c that a visual test is necessary, controller 51 sets annotative information or the like indicating a ground for the determination that a visual test is necessary, as the rule comment with rule 52c. For example, when there is a large difference between the analysis value of the analysis item for occult blood obtained by urine qualitative analyzer 20 and the analysis value of the analysis item for red blood cell obtained by urinary sediment analyzer 30, controller 51 sets, as the rule comment, annotative information "due to a large difference between the occult-blood value obtained by the urine qualitative analyzer and the red-blood-cell value obtained by the urinary sediment analyzer", which has been set in rule 52c.

Also, when determining with rule 52c that a predetermined relation exists between the analysis values of at least two analysis items on the target sample, controller 51 sets annotative information on a disease judged from the predetermined relation as the rule comment with rule 52c. For example, when the ratio of albumin and creatinine obtained by urine qualitative analyzer 20 is greater than a predetermined value and also the analysis value of cast concentration obtained by urinary sediment analyzer 30 is greater than a predetermined value, controller 51 sets annotative information "suspicion of early diabetic nephropathy", which has been set in rule 52c, as the rule comment. When the analysis value of protein concentration obtained by urine qualitative analyzer 20 is greater than a predetermined value and also the analysis value of red blood cell obtained by urinary sediment analyzer 30 is greater than a predetermined value, controller 51 sets annotative information "suspicion of chronic nephritis", which has been set in rule 52c, as the rule comment.

Moreover, controller 51 compares the result of the analysis by urine qualitative analyzer 20 and the result of the analysis by urinary sediment analyzer 30 with rule 52c to perform a cross-check for checking discrepancy between the analysis results. Controller 51 performs the cross-check with rule 52c and sets the result ofthe cross-check as the rule comment.

Controller 51 sets the review flags, the high/low flags, the analysis rule comments, the visual test flag, and the rule comment at predetermined timing. For example, controller 51 makes the determinations with rule 52c and sets the flags and the annotative information as the above-mentioned values when the analysis values of all the analysis items processed for the sample are stored into the analysis result table. In a case where pieces of annotative information are generated, the controller 51 stores the pieces of annotative information as the rule comment.

The visual test result is the result of a visual test on each test item inputted through input unit 54. The visual-test-result input date and time is the date and time when the visual test result was saved to the analysis result table. The report comment is a comment on the visual test inputted by the operator during the visual test.

Note that the tables set in database 52b are not limited to the configurations illustrated in Figs. 4A to 4D. The tables may only need such that database 52b can store information on patients, information on samples, information on measurement orders, and information on analysis results.

Next, detailed configurations of screens 100, 200, displayed on display unit 53 of information processing apparatus 50, are described. The display of the screens 100, 200, and the switching of the display contents of screens 100, 200 are all done by controller 51 executing program 52a.

As illustrated in Fig. 5, screen 200 includes sample list display region 210 disposed at the center and visual-test-result input button 220 disposed at the top. Screen 200 is displayed on display unit 53 when list display button 101, illustrated in Fig. 6 to be mentioned later, is pressed through input unit 54.

Sample list display region 210 displays information on each sample in list form based on the information read from each table in database 52b. Sample list display region 210 includes items such as "sample ID", "qualitative analysis" indicating the status of the urine qualitative analysis result, "sediment analysis" indicating the status of the urinary sediment analysis result, "visual test" indicating the status of the visual test result, "body fluid" indicating the status of the body-fluid analysis result, "body-fluid visual test" indicating the status of the body-fluid visual test result, "patient ID", and "patient name" in this order from the left. The horizontal area within which to display the items and the vertical area within which to display the pieces of sample information can be shifted with scroll operation parts disposed to the right and under sample list display region 210.

A check mark under "qualitative analysis" indicates a state where the result of a urine qualitative analysis has been stored in the analysis result table in database 52b. A check mark under "sediment analysis" indicates a state where the result of a urinary sediment analysis has been stored in the analysis result table in database 52b. A check mark under "visual test" indicates a state where the result of a visual test on the urine sample has been stored in the analysis result table in database 52b. A circle mark under "visual test" indicates a state where a visual test has been determined to be necessary for the urine sample, and a flag indicating that a visual test is necessary has been set as the visual test flag in the analysis result table.

A check mark under "body fluid" indicates a state where the result of an analysis on the body fluid sample by urinary sediment analyzer 30 has been stored in the analysis result table in database 52b. A check mark under "body-fluid visual test" indicates a state where the result of a visual test on the body fluid sample has been stored in the analysis result table in database 52b. A circle mark under "body-fluid visual test" indicates a state where a visual test has been determined to be necessary for the body fluid sample, and a flag indicating that a visual test is necessary has been set as the visual test flag in the analysis result table.

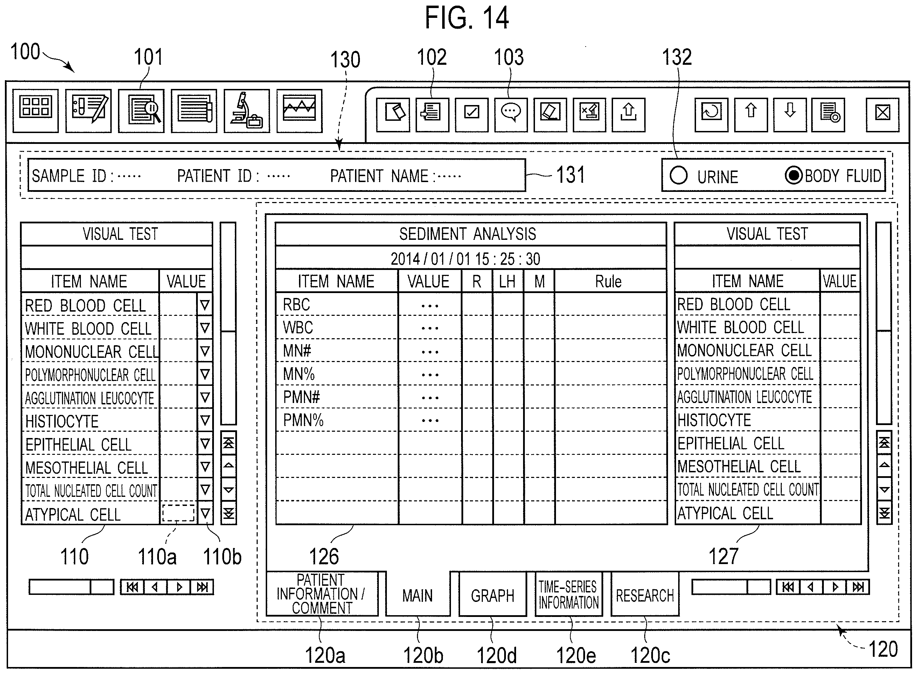

When a sample with a circle mark under "visual test" is selected and visual-test-result input button 220 is pressed in this state through input unit 54, screen 100 including information on the urine sample selected from sample list display region 210 is displayed on display unit 53. In this case, screen 100 as illustrated in Fig. 6 is displayed. On the other hand, when a sample with a circle mark under "body-fluid visual test" is selected and visual-test-result input button 220 is pressed in this state through input unit 54, screen 100 including information on the body-fluid sample selected from sample list display region 210 is displayed on display unit 53. In this case, screen 100 as illustrated in Fig. 14 is displayed.

Screen 100 illustrated in Fig. 6 includes input-value display region 110, reference-information display region 120, header-information display region 130, list display button 101, save button 102, and comment input button 103.

Header-information display region 130 includes regions 131, 132. Region 131 displays the sample ID, the patient ID, and the patient name according to the information read from the patient information table. Region 132 displays which one of a urine sample or a body-fluid sample is the sample displayed on screen 100, on the basis of the information read from the measurement order table. Region 132 illustrated in Fig. 6 indicates that the information on the sample displayed on screen 100 is about a urine sample.

Input-value display region 110 displays test items for a visual test under which the count values of particles counted in the visual test can be inputted for the sample displayed on screen 100. The test items refer to the types of particles to be tested. The test items for the visual test displayed in input-value display region 110 include test items for testing the same types of particles as the analysis items for urinary sediment analyzer 30, sub-classified items that are more detailed than the analysis items for urinary sediment analyzer 30, and the like.

The test items for testing the same types of particles as the analysis items for urinary sediment analyzer 30 include, for example, a test item "red blood cell" for testing the red blood cells which have been analyzed under the analysis item "RBC" by urinary sediment analyzer 30. In this way, when there is discrepancy between the value of the analysis item "OB" obtained by urine qualitative analyzer 20 and the value of the analysis item "RBC" obtained by urinary sediment analyzer 30, the operator can visually check and count the red blood cells and report the count value of the test item "red blood cell" to a doctor.

Also, the sub-classified items that are more detailed than the analysis items for urinary sediment analyzer 30 include, for example, test items "hyaline cast", "epithelial cast", and "granular cast" and the like, which are obtained by sub-classifying the cast that has been analyzed under the analysis item "CAST" by urinary sediment analyzer 30. In this way, when the value of the analysis item "PRO" detected by urine qualitative analyzer 20 is high and the value of the analysis item "CAST" analyzed by urinary sediment analyzer 30 in a retest is high as well, the operator can visually check, sub-classify, and count the casts and report the count values of the test items "hyaline cast", "epithelial cast", and "granular cast" to a doctor.

For each test item for the visual test, input-value display region 110 includes value display region 110a for displaying the count values obtained in the visual test, and button 110b. When inputting the count values obtained in the visual test, the operator uses input unit 54 to press button 110b for the corresponding visual test item to be inputted and display a window for this visual test item.

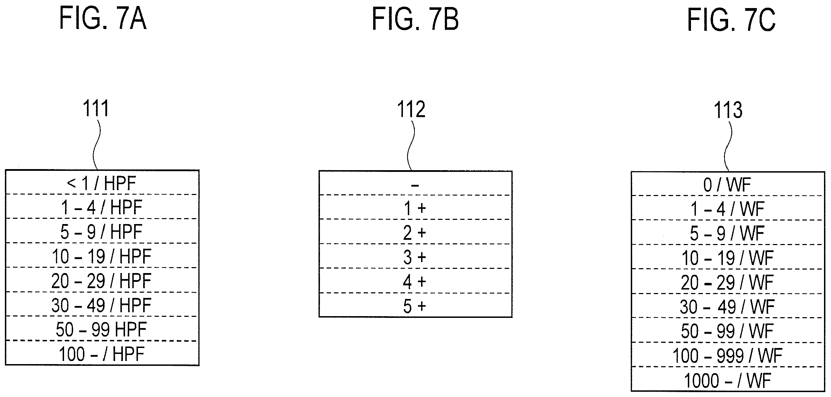

For example, when any of buttons 110b for the test items for visually testing blood cells and epithelial cells is pressed through input unit 54, window 111 illustrated in Fig. 7A is displayed under that button 110b. Window 111 is designed such that any one of less than 1, 1 to 4, 5 to 9, 10 to 19,20 to 29, 30 to 49, 50 to 99, more than 100 can be selected through input unit 54 as the count within a field. For example, when button 110b for an item for visually testing a cast is pressed through input unit 54, window 112 illustrated in Fig. 7B or window 113 illustrated in Fig. 7C is displayed under that button 110b. Window 112 is designed such that any one of "-", "1+", "2+", "3+", "4+", and "5+" can be selected through input unit 54 in accordance with the count. Window 113 is designed such that any one of 0, 1 to 4, 5 to 9, 10 to 19, 20 to 29, 30 to 49, 50 to 99, 100 to 999, and more than 1000 can be selected through input unit 54 as the count within the whole field.

The operator selects the count value obtained in the visual test by pressing an item in one of windows 111 to 113 as illustrated in Figs. 7A to 7C that matches the result of the counting ofthe particle in the visual test, through input unit 54. As a result, the one of windows 111 to 113 is closed, and the display of corresponding value display region 110a in input-value display region 110 is updated to the count value inputted through input unit 54.

To save the visual test results inputted in input-value display region 110, the operator presses save button 102, which is at the top of screen 100, through input unit 54. Consequently, the count values displayed in input-value display region 110 are stored as visual test results in the analysis result table. In addition, the visual test flag in the analysis result table is changed from the flag indicating that a visual test is necessary to a flag indicating that no visual test is necessary. As a result, when screen 200 is displayed, a check mark is displayed under the item "visual test".

Note that the operator's command to save the count values obtained in the visual test to the analysis result table is not limited to operating save button 102 through input unit 54. For example, program 52a may be constructed to display a dialogue such as "Do you want to save the count values obtained in the visual test?" when closing screen 100, and the count values obtained in the visual test may be saved to the analysis result table when the operator selects a "Yes" button displayed in the dialogue through input unit 54.

Note that the items displayed in input-value display region 110 can be changed by operating a setting screen not illustrated through input unit 54. Moreover, shortcut keys are set on input unit 54 to make it easier to input visual test results through input unit 54 while performing a visual test with microscope 60. For example, input-value display region 110 is designed such that inputting a key "1" into the keyboard of input unit 54 selects the test item for red blood cell and opens an input window as described above with reference to Figs. 7A to 7C. In this way, visual test results can be inputted efficiently. Furthermore, when a test item for the visual test is selected, information processing apparatus 50 makes a sound corresponding to the selected test item. In this way, it is possible to check the selected test item for the visual test not only through screen 100 but also through a sound and thus prevent a wrong test item from being selected for the visual test.

Reference-information display region 120 includes switch parts 120a to 120e. Switch parts 120a to 120e receive a switch command to switch the display content of reference-information display region 120. Upon receipt of a switch command at one of switch parts 120a to 120eb through input unit 54, controller 51 displays a display content corresponding to the switch command in reference-information display region 120. Specifically, reference-information display region 120 is formed of a so-called tab control, and switch parts 120a to 120e are formed of buttons that switch the tab display. When switch parts 120a to 120e are pressed through input unit 54, the display content of reference-information display region 120 are changed as illustrated in Figs. 6, 8, 10, 11, and 12, respectively. Note that reference-information display region 120 does not necessarily have to be formed of a tab control, but other means may be used for the display and switching of reference-information display region 120.

Reference-information display region 120 shown in Fig. 6 includes patient-information display region 141, sample-information display region 142, report-comment display region 143, review-comment display region 144, research-information display region 145, and rule-comment display region 146.

Patient-information display region 141 displays attribute information on the patient from whom the sample has been collected, on the basis of the information read from the patient information table. Patient-information display region 141 displays the patient name, patient ID, sex, age, blood type, and the like, for example. Sample-information display region 142 displays information on the sample according to the information read from the sample information table. Sample-information display region 142 displays the sample ID, reception date, reception number, clinical department, ward name, and the like, for example.

Report-comment display region 143 displays a comment on the visual test on the sample received from the operator, on the basis of the information read from the report comment in the analysis result table. Controller 51 receives input of the comment on the visual test on the sample. Specifically, when comment input button 103 is pressed through input unit 54, controller 51 displays a comment input window and receives input of a comment. When the comment input window is closed, controller 51 updates the display content of report-comment display region 143 to the received comment. When save button 102 is pressed through input unit 54, controller 51 saves the comment displayed in report-comment display region 143 as the report comment in analysis result table.

In this way, the operator can, for example, leave a comment such as "A follow-up might be necessary." for the doctor in charge. The operator can also leave an opinion based on his or her visual observation in report-comment display region 143, and can leave an opinion such as "many pathological casts" or "severe hemolyzation".

Review-comment display region 144 displays annotative information on the results of the analyses on the sample generated by the analyzers, on the basis of the review comment read from the analysis result table. For example, review-comment display region 144 displays "abnormality in RBC-X'TAL fractionation" or the like. By recognizing the "abnormality in RBC-XTAL fractionation" displayed in review-comment display region 144, the operator can notice the possibility of a failure to properly automatically classify the red blood cells and the crystals. Thus, the operator can perform a visual test by focusing on the red blood cells and the crystals.

Research-information display region 145 displays annotative information on the results of the analyses on the sample generated by the analyzers, on the basis of the research information read from the analysis result table. For example, in a case where urinary sediment analyzer 30 has obtained an analysis result indicating the presence of many renal-tubular epithelial cells and an analysis result indicating unevenness in the form of the red blood cells, research-information display region 145 displays annotative information such as "suspicion of a kidney disease".

As mentioned above, annotative information is set as the research information when a predetermined relation exists between the analysis values of at least two analysis items. Thus, when a predetermined relation exists between the analysis values of at least two analysis items, research-information display region 145 displays annotative information on a disease judged from the predetermined relation. In the case where research-information display region 145 displays "suspicion of a kidney disease", the operator can check the presence of renal-tubular epithelial cells, the form of the red blood cells, and the like in a visual test. Instead of "suspicion of a kidney disease", research-information display region 145 may display "unevenness in the form of the red blood cells", for example. In this way, focusing on the red blood cells, the operator can visually check whether or not the form of the red blood cells is actually uneven.

Rule-comment display region 146 displays annotative information on the sample generated by controller 51, on the basis of the rule comment read from the analysis result table. When a visual test is necessary, rule-comment display region 146 displays, for example, "due to a large difference between the occult-blood value obtained by the urine qualitative analyzer and the red-blood-cell value obtained by the urinary sediment analyzer" or the like as a ground for the determination that a visual test is necessary. When a predetermined relation exists between the analysis values of at least two analysis items, rule-comment display region 146 displays, for example, "suspicion of early diabetic nephropathy", "suspicion of chronic nephritis", or the like. Rule-comment display region 146 displays the result of the cross-check performed between the result of the analysis by urine qualitative analyzer 20 and the result of the analysis by urinary sediment analyzer 30.

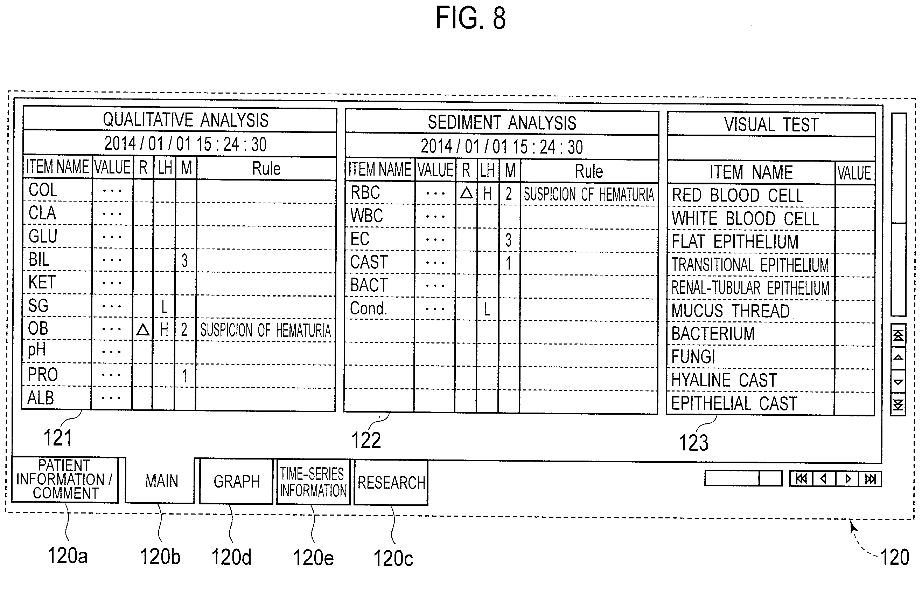

Reference-information display region 120 illustrated in Fig. 8 includes regions 121 to 123, as described with reference to Fig. 1. Region 121 displays the analysis values of urine-qualitative-analysis items and the like. Region 122 displays the analysis values of urinary-sediment-analysis items and the like. Region 123 displays the test items for a visual test similar to those in input-value display region 110, and visual test results read from the analysis result table in database 52b, stored in storage 52, in relation to the sample displayed on screen 100. Regions 121, 122 are displayed according to the measurement date and time, the analysis values, and the like read from the analysis result table, while region 123 is displayed according to the visual test results read from the analysis result table. The horizontal area within which to display the items and the vertical area within which to display the items can be shifted with scroll operation parts disposed to the right and under sample list display region 210.

In the case where reference-information display region 120 includes region 123 as illustrated in Fig. 8 too, the count values displayed in input-value display region 110 are stored as visual test results in the analysis result table when save button 102, described above reference to Fig. 6, is pressed through input unit 54. In this case, further, the visual test results are read from the analysis result table, and the visual-test count value in region 123 illustrated in Fig. 8 are updated.

The operator can easily check discrepancy between the result of the urine qualitative analysis and the result of the urinary sediment analysis when the analysis value of each urine-qualitative-analysis item and the analysis value of each urinary-sediment-analysis item are displayed on single screen 100, as illustrated in Fig. 8. That is, by referring to screen 100, the operator can compare the result of the urine qualitative analysis and the result of the urinary sediment analysis and easily perform a cross-check for checking discrepancy between the analysis results. Hence, the operator can efficiently perform a visual test based on discrepancy between the analysis results.

The analysis values of the analysis items in each region 121, 122 are arranged next to each other vertically, and the count values of the test items in region 123 are arranged next to each other vertically. Also, regions 121 to 123 are arranged next to each other horizontally. Displaying regions 121 to 123 in this fashion makes it easy to view and compare the analysis values of the analysis items and the count values of the test items with each other horizontally.

The analysis values of the analysis items in each region 121, 122 may be arranged next to each other horizontally, and the count values of the test items in region 123 may be arranged next to each other horizontally. In this case, with regions 121 to 123 arranged next to each other vertically, it is easy to view and compare the analysis values of the analysis items and the count values of the test items with each other vertically. Meanwhile, it is in particular preferable to display regions 121 to 123 like Fig. 8 in the case where the analysis items for the analyzers and the test items for the visual test are large in number as in this embodiment.

Note that in the case where both urine qualitative analyzer 20 and urinary sediment analyzer 30 have analyzed the target sample, both regions 121, 122 are displayed in reference-information display region 120, as illustrated in Fig. 8. In the case where only one of urine qualitative analyzer 20 and urinary sediment analyzer 30 has analyzed the target sample, only the region corresponding to the analyzer that has performed the analysis is displayed in reference-information display region 120.

The analysis items in region 121, the analysis items in region 122, and the test items in region 123 can be rearranged. The operator can rearrange the analysis items and the test items that are arranged vertically in regions 121 to 123, by operating a rearrangement window not illustrated through input unit 54. In this way, the operator can display each horizontally adjacent two of regions 121 to 123 such that analysis items and test items in these two regions related to each other are arranged next to each other horizontally.

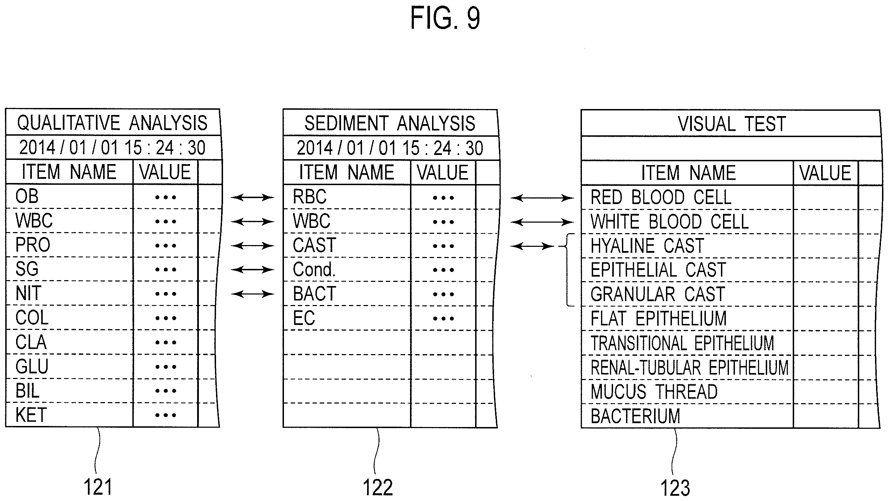

As illustrated in Fig. 9, the operator can, for example, arrange "OB" in region 121, "RBC" in region 122, and "red blood cell" in region 123 next to each other horizontally. "OB" is an analysis item for occult blood, and "RBC" is an analysis item for red blood cell. Thus, "OB", "RBC", and "red blood cell", arranged next to each other horizontally, are related to each other. Also, the operator can arrange "WBC" in region 121, "WBC" in region 122, and "white blood cell" in region 123 next to each other horizontally. Each "WBC" is an analysis item for white blood cell. Thus, "WBC", "WBC", and "white blood cell", arranged next to each other horizontally, are related to each other.

Also, the operator can arrange "PRO" in region 121, "CAST" in region 122, and "hyaline cast", "epithelial cast", or "granular cast" in region 123 next to each other horizontally. "PRO" is an analysis item for protein concentration, and "CAST" is an analysis item for cast. Thus, "PRO", "CAST", and "hyaline cast", arranged next to each other horizontally, are related to each other. Also, the operator can arrange "SG" in region 121 and "Cond." in region 122 next to each other horizontally. "SG" is an analysis item for specific gravity, and "Cond." is an analysis item for electric conductivity. Thus, "SG" and "Cond.", arranged next to each other horizontally, are related to each other. Also, the operator can arrange "NIT" in region 121 and "BACT" in region 122 next to each other horizontally. "NIT" is an analysis item for nitrite, and "BACT" is an analysis item for a bacterium. Thus, "NIT" and "BACT", arranged next to each other horizontally, are related to each other.

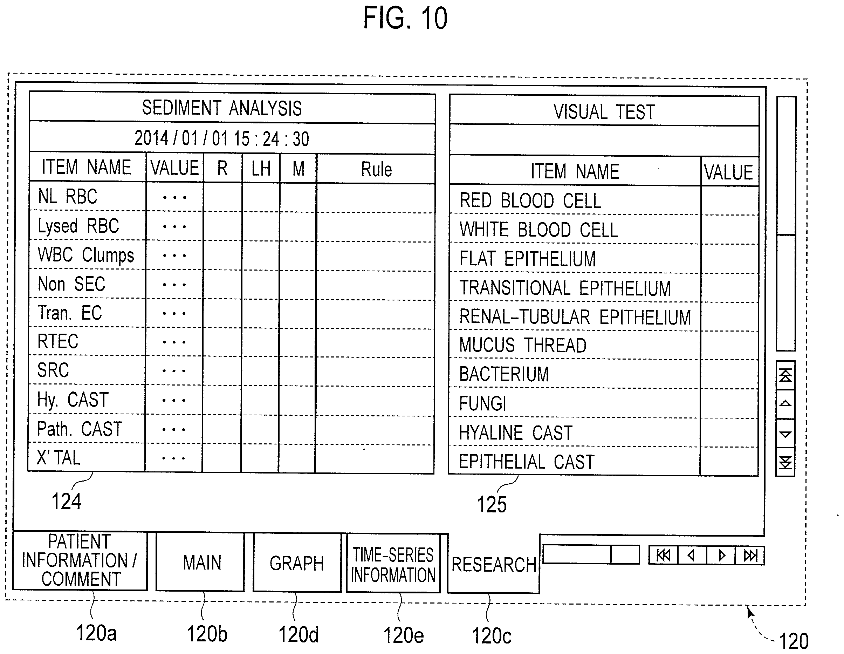

Note that regions 121, 122 illustrated in Fig. 8 display the analysis values of analysis items that can be considered useful from a clinical point of view. Also, region 124 illustrated in Fig. 10 to be mentioned later display the analysis values of analysis items that can be considered useful from a research point of view. In a case of displaying the analysis values of all the analysis items illustrated in Figs. 8 and 10 on single screen 100, the combinations of related analysis items and test items can possibly include combinations other than those mentioned above.

Since the analysis items and the test items in regions 121 to 123 can be rearranged as described above, the operator can display the analysis items and the test items in two regions such that analysis items and test items in these two regions related to each other are arranged next to each other horizontally, as illustrated in Fig. 9. Discrepancy between the result of the urine qualitative analysis and the result of the urinary sediment analysis can be checked more easily when the related analysis items and test items are arranged next to each other horizontally, as illustrated in Fig. 9, than when the analysis items and the test items are arranged as illustrated in Fig. 8. Thus, the operator can perform a visual test more efficiently, basing the discrepancy between the analysis results.

In addition to the manual rearrangement of the analysis items in regions 121, 122 and the test items in region 123 by the operator as described above, controller 51 also automatically rearranges the analysis items in regions 121, 122 and the test items in region 123 as illustrated in Fig. 9 upon pressing of a button or the like through input unit 54.

Referring back to Fig. 8, regions 121, 122 include items such as "R", "LH", "M", and "Rule" in addition to the items "item name" and "value". The items "R", "LH", "M", and "Rule" are displayed based respectively on the review flag, high/low flag, analyzer flag, and analysis rule comment read from the analysis result table.

For regions 121, 122 illustrated in Fig. 8, "OB" and "RBC" are such that: the item "R" displays "Δ" indicating that controller 51 has determined that retesting is necessary; the item "LH" displays "H" indicating that the analysis result is a high value; the item "M" displays "2" indicating that qualitative analysis controller 21 or sediment analysis controller 31 has determined that retesting is necessary; and the item "Rule" displays "suspicion of hematuria". "SG" and "Cond." are such that the item "LH" display "L" indicating that the analysis result is a low value. "PRO" and "CAST" are such that the item "M" displays "1" indicating an abnormality in the analysis. "BIL" and "EC" are such that the item "M" displays "3" indicating low reliability.

As described above, regions 121, 122 display a large amount of information, as illustrated in Fig. 8. Thus, the operator can efficiently perform a visual test by referring to these items.

When an analysis item with "Δ" and "2", indicating that retesting is necessary, is present on the screen, the operator can perform a visual test by focusing on the analysis item with those marks. This is described by taking an example where urine qualitative analyzer 20 has detected urine occult blood. When urine occult blood is detected, thereby suggesting the presence of red blood cells in the urine sample, the subject is suspected to have acute glomerulonephritis, pyelonephritis, cystitis, urethritis, renal tumor, renal concretion, or the like. Thus, to check the red blood cells in the urine sample, urinary sediment analyzer 30 also tests the urine sample. Here, even if the urinary sediment test detects only few red blood cells, it is still not clear whether the urine sample has no abnormality or the result of the urinary sediment test is not accurate. For this reason, the operator needs to visually check the red blood cells. In this case, in regions 121, 122 illustrated in Fig. 8, "Δ", indicating that retesting is necessary, is displayed in association with the analysis item for occult blood and the analysis item for red blood cell. Hence, the operator can easily understand that he or she needs to perform a visual test by focusing on the red blood cells. The operator can then carefully perform a visual test to check the red blood cells in the urine sample, and input the accurate number of red blood cells and input a comment on the severity of hemolyzation of the red blood cells. Consequently, accurate information on the state ofthe urine sample can be reported to the doctor in charge.

Also, when an analysis item with "3", indicating low reliability, is present, it suggests that urine qualitative analyzer 20 or urinary sediment analyzer 30 has failed to obtain an accurate analysis result for that analysis item. The operator can then perform a visual test by focusing on the analysis item with that mark. For example, when "CAST" is given "3", indicating low reliability, the operator can visually check whether mucus threads with similar shapes to casts are present in the sample.

In another example, when "CAST" is given "H", indicating that the analysis result is a high value, the operator can perform a visual test by focusing on the casts and visually check the forms of the casts, the numbers of casts present, and the like. This is described by taking an example where urine qualitative analyzer 20 has detected urine protein. When urine protein is detected, there is a suspicion of chronic nephritis, glomerulonephritis, pyelonephritis, or the like. Thus, to estimate the extent of progression of the disease and its area, urinary sediment analyzer 30 also tests the urine sample, and the presence of casts and their amounts are checked from the result of the urinary sediment test obtained. In a case where the disease has progressed, pathological casts (cellular casts) other than hyaline casts are often found in the urine such as "red-blood-cell casts" and "white-blood-cell casts", which are red blood cells and white blood cells leaking from glomeruli and turned into cast forms together with protein components at the renal tubule. When such pathological casts are present, it is possible to determine that glomerulonephritis is likely to have occurred. When "CAST" is given "H", indicating that the analysis result is a high value, the operator can carefully perform a visual test by focusing on the casts. By observing the forms of the casts, their amounts, and the like, the operator can input a comment such as "pathological casts have been observed" or "possibly glomerulonephritis?". Consequently, accurate information on the state ofthe urine sample can be reported to the doctor in charge.

Reference-information display region 120 illustrated in Fig. 10 includes regions 124 and 125. Region 124 displays the analysis values of analysis items that are useful from a research point of view, among the urinary-sediment-analysis items. Region 124 is displayed according to the measurement date and time and analysis values read from the analysis result table. Region 125 is similar to region 123 in Fig. 8.

Region 124 also includes items such as "R", "LH", "M", and "Rule" as in region 122 illustrated in Fig. 8. Note that all the urine-qualitative-analysis items are considered useful from a clinical point of view; thus, the urine-qualitative-analysis items are not displayed in reference-information display region 120 illustrated in Fig. 10. In the case illustrated in Fig. 10 too, the operator can refer to, as reference information, the analysis values of the analysis items that are useful from a research point of view, and thus perform a visual test efficiently.

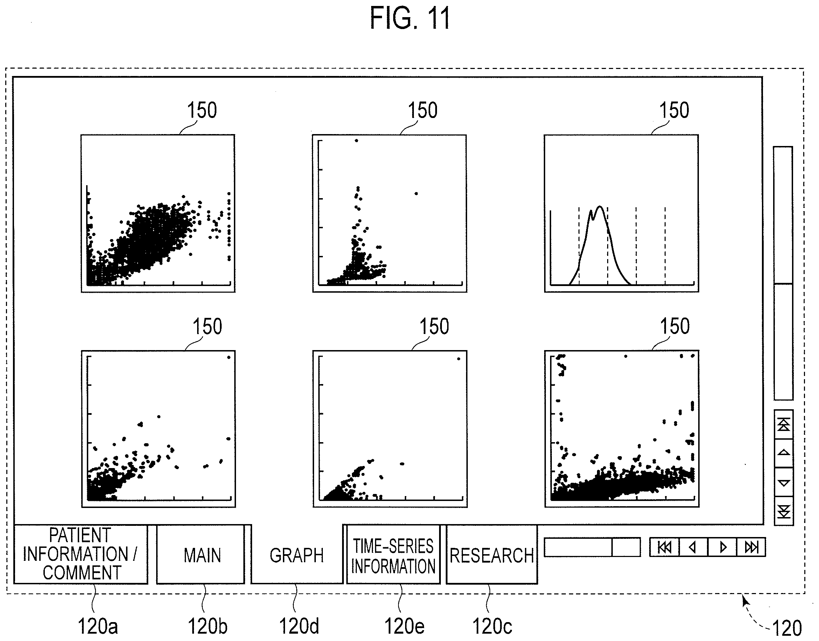

Reference-information display region 120 illustrated in Fig. 11 displays graphs 150 such as scattergrams or histograms based on the graphs read from the analysis result table. In the example illustrated in Fig. 11, graphs 150 are graphs used when the analysis result is generated by urinary sediment analyzer 30.

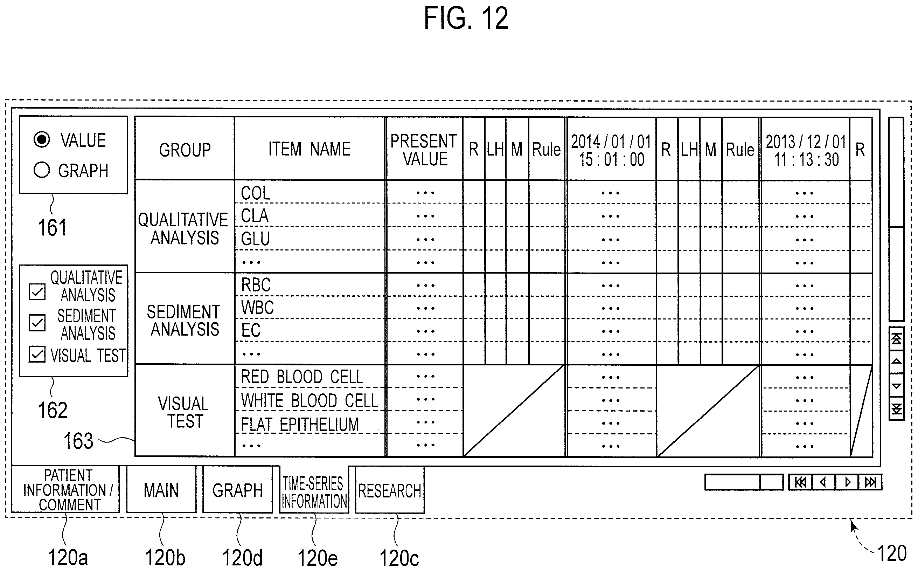

Reference-information display region 120 illustrated in Fig. 12 include regions 161 to 163. Region 161 receives a setting as to which of the analysis values and the graphs are to be displayed in region 163. Assume that the radio button in region 161 for displaying the analysis values is selected. In this case, on the basis of measurement dates and times, analysis values, visual test results, visual-test-result input dates and times read from the analysis result table, region 163 displays the analysis values of the analysis items and the visual test results in time-series order, as illustrated in Fig. 12. In Fig. 12, in total three results are displayed including the present result, the immediately preceding result, and the second preceding result. Older results can be displayed with a scroll operation part disposed under reference-information display region 120.

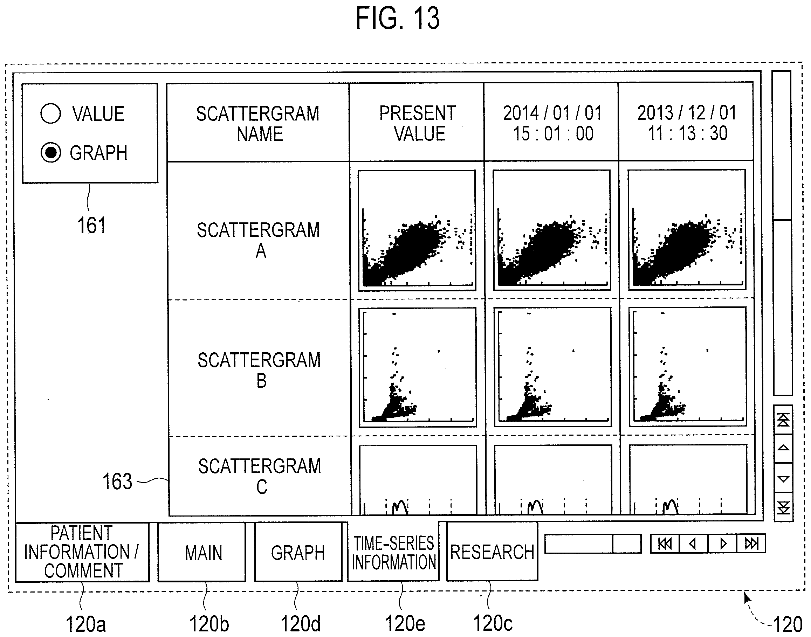

Region 162 receives a setting as to which one or ones of the qualitative analysis result, the sediment analysis result, and the visual test result is or are to be displayed in region 163 illustrated in Fig. 12. Assume that the radio button in region 161 for displaying the graphs is selected. In this case, on the basis of the measurement dates and times and graphs read from the analysis result table, region 163 displays the graphs such as scattergrams or histograms in time-series order, as illustrated in Fig. 13.

Reference-information display region 120 illustrated in Fig. 14 includes regions 126 and 127. Region 126 displays the analysis values of analysis items for the body fluid sample according to the measurement date and time and analysis values read from the analysis result table. Input-value display region 110 and region 127 display the visual test items for the body fluid sample. Note that, as in the case of the urine sample, the display content of reference-information display region 120 are switched also for the body fluid sample by switch parts 120a to 120e.

With reference-information display region 120 displayed as illustrated in Figs. 6 to 14, the operator can efficiently perform a visual test by referring to reference-information display region 120. Moreover, the operator can change the display content of reference-information display region 120 as illustrated in Figs. 6, 8, and 10 to 14 by pressing switch parts 120a to 120e through input unit 54. In this way, the operator can switch the display content of reference-information display region 120 with a simple operation and efficiently perform a visual test.

Next, processing performed by controller 51 is described with reference to flowcharts.

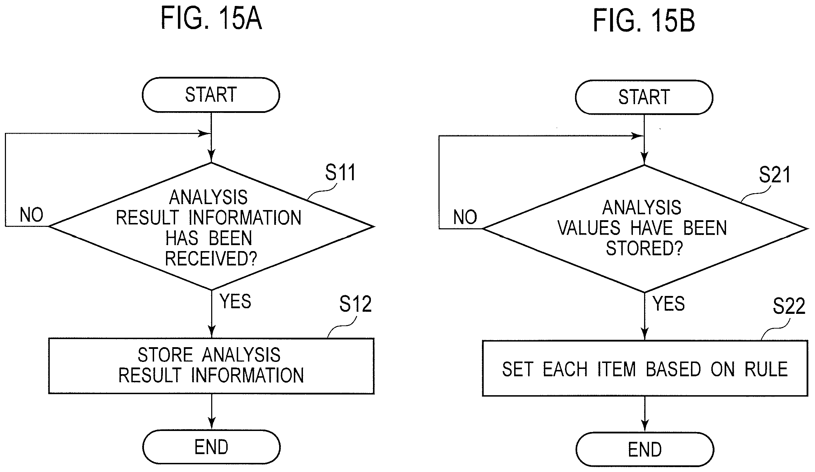

As illustrated in Fig. 15A, in Step S11, controller 51 determines whether or not analysis result information has been received from urine qualitative analyzer 20 or urinary sediment analyzer 30. If analysis result information has been received, controller 51 stores the received analysis result information in the analysis result table in Step S12.

As illustrated in Fig. 15B, in Step S21, controller 51 determines whether or not all the analysis values of the analysis items processed for the sample have been stored in the analysis result table. If all the analysis values have been stored, controller 51 sets each item in the analysis result table with rule 52c in Step S22. Specifically, controller 51 sets the items of review flag, high/low flag, analysis rule comment, visual test flag, and rule comment for the corresponding sample. Controller 51 may set each item in the analysis result table with rule 52c at predetermined timing other than when all the analysis values are stored.

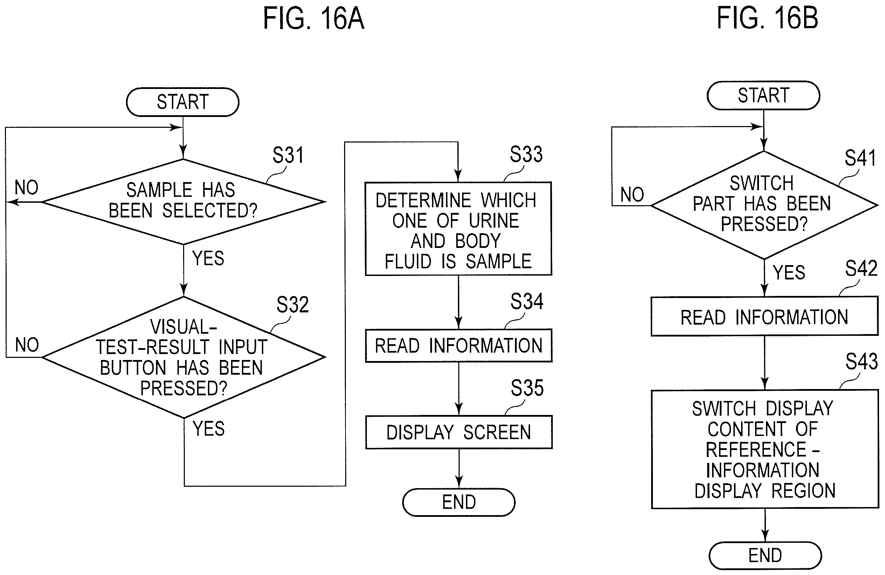

As illustrated in Fig. 16A, in Step S31, controller 51 determines whether or not a sample has been selected in sample list display region 210 in screen 200. In Step S32, controller 51 determines whether or not visual-test-result input button 220 has been pressed through input unit 54. If visual-test-result input button 220 has been pressed through input unit 54 with the sample selected in sample list display region 210, controller 51 determines in step S33 which one of urine and body fluid is the selected sample. In Step S34, on the basis of the selected sample, controller 51 reads information necessary for displaying screen 100 illustrated in Fig. 6 or Fig. 14 from database 52b. In Step S35, controller 51 displays screen 100 as illustrated in Fig. 6 or Fig. 14 on display 53 according to the sample type acquired in Step S33 and the information read in Step S34.

As illustrated in Fig. 16B, in Step S41, controller 51 determines whether or not any of switch parts 120a to 120e has been pressed through input unit 54 and a switch command has been received in screen 100. If a switch command has been received, controller 51 reads information necessary for displaying reference-information display region 120 corresponding to the content of the switch command from database 52b in Step S42. In Step S43, controller 51 switches the display content of reference-information display region 120 according to the information read in Step S42. For example, if switch part 120b has been pressed through input unit 54, controller 51 reads the results of the analyses by urine qualitative analyzer 20 and urinary sediment analyzer 30, the result of a visual test, and the like from the analysis result table, and displays the read analysis results, visual test result, and the like within reference-information display region 120.

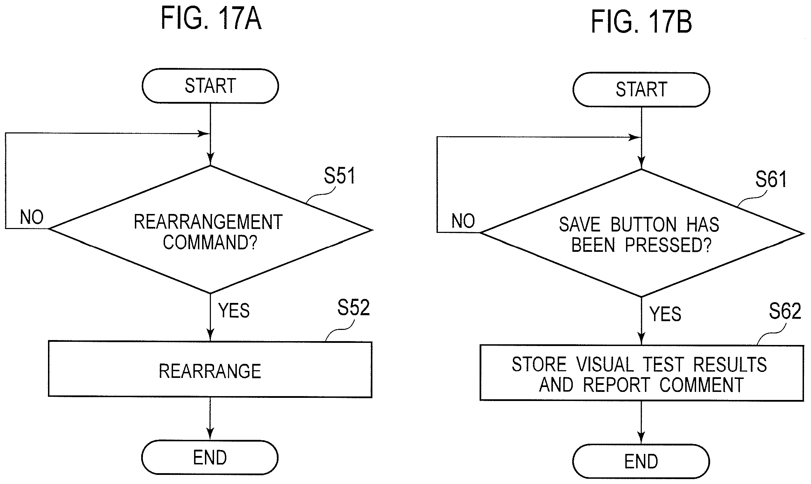

As illustrated in Fig. 17A, in the case where the display content of reference-information display region 120 is as illustrated in Fig. 8, controller 51 determines in Step S51 whether or not the button or the like for rearranging the analysis items in regions 121, 122 and the test items in region 123 has been pressed through input unit 54. If the button or the like for rearrangement has been pressed through input unit 54, controller 51 rearranges the analysis items in regions 121, 122 and the test items in region 123 as illustrated in Fig. 9 in Step S52. Note that controller 51 also performs similar processing for rearranging analysis items and test items in the case where the display content of reference-information display region 120 is as illustrated in Fig. 14.

As illustrated in Fig. 17B, in Step S61, controller 51 determines whether or not save button 102 on screen 100 has been pressed through input unit 54. If save button 102 has been pressed through input unit 54, controller 51 stores the count values displayed in input-value display region 110 into the analysis result table as the visual test results in Step S62. If region 123 is being displayed in reference-information display region 120, controller 51 also reads the visual test results from the analysis result table and updates the visual-test count values in a visual test in region 123. Also, in the case where a comment is displayed in report-comment display region 143, as illustrated in Fig. 6, when save button 102 is pressed through input unit 54, controller 51 stores the comment displayed in report-comment display region 143 into the analysis result table.

## Claims

1. A sample testing system (10) comprising:
a sample analyzer that analyzes (30) a particle in a sample; and
an information processing apparatus (50) that receives a result of an analysis on the sample received from the sample analyzer (30), wherein
the information processing apparatus (50) comprises a controller (51), a display unit (53), and an input unit (54),
the sample testing system (10) **characterized by**:
- the controller (51) controls the display unit (53) to display a screen including a reference-information display region (120) for the result of the analysis on the sample, received from the sample analyzer (20, 30), and an input-value display region (110) for a visual test to be performed on the sample with a microscope (60), wherein
when the controller (51) has determined that retesting is necessary, then the controller (51) controls the display unit (53) to indicate in the reference-information display region (120) that the controller has determined that retesting is necessary, and
- with the screen displayed, the controller (51) receives a count value of the particle counted in the visual test on the sample, through the input unit (54), and controls the display unit (53) to display the received count value in the input-value display region (110).

2. The sample testing system (10) according to claim 1, wherein
the sample analyzer (30) analyzes the sample in terms of analysis items related to the particle,
the reference-information display region (120) displays analysis values of the respective analysis items as the result of the analysis on the sample,
the input-value display region (110) displays test items and value display regions for the respective test items, the test items including sub-classified items that are more detailed than the analysis items, and
the controller (51) receives, through the input unit (54), the count value of the particle counted in the visual test for each of the test items and displays the received count values in the value display regions.

3. The sample testing system (10) according to claim 2, wherein when the analysis value of any of the analysis items for the sample represents low reliability or abnormality, the controller (51) displays information indicating the low reliability or the abnormality, in association with the analysis item.

4. The sample testing system (10) according to any one of claims 1 to 3, further comprising a second sample analyzer (20) that analyzes the sample with a measurement principle different from that of the sample analyzer (30), wherein
the sample analyzer (30) analyzes the sample as a retest after the second sample analyzer (20, ) analyzes the sample, and
the controller (51) further displays a result of an analysis on the sample received from the second sample analyzer (20,) in the reference-information display region (120).

5. The sample testing system (10) according to claim 4, wherein the controller (51) displays a first region (122) and a second region (121) next to each other within the reference-information display region (120), the first region (122) including the result of the analysis by the sample analyzer (30), the second region (121) including the result of the analysis by the second sample analyzer (30).

6. The sample testing system (10) according to claim 4 or 5, wherein
the controller (51) displays a first region (122) and a third region (123) next to each other within the reference-information display region, the first region (121, 122) including the result of the analysis by the sample analyzer (30), the third region (123) including a result of the visual test on the sample, and
when receiving a predetermined command with the count value displayed in the input-value display region (110), the controller displays the count value displayed in the input-value display region (110), as the result of the visual test in the third region (123).

7. The sample testing system (10) according to any one of claims 4 to 6, wherein the sample analyzer (30) is a urinary sediment analyzer (30), and the second sample analyzer is a urine qualitative analyzer (20).

8. The sample testing system (10) according to claim 7, wherein the controller (51) displays analysis items in a first region (122) and a second region (121) within the reference-information display region (120) such that the analysis items in the first region (122) and the second region (121) related to each other are arranged next to each other horizontally, the first region (122) including the result of the analysis by the urinary sediment analyzer (30), the second region (121) including the result of the analysis by the urine qualitative analyzer (20).

9. The sample testing system (10) according to claim 8, wherein the related analysis items in the first region (122) and the second region (121) are one combination selected from a group of combinations of occult blood and red blood cell, protein concentration and cast, nitrite and bacterium, and specific gravity and electric conductivity.

10. The sample testing system (10) according to any one of claims 7 to 9, wherein the controller (51) performs a cross-check between the result of the analysis by the urine qualitative analyzer (20) and the result of the analysis by the urinary sediment analyzer (30), and displays a result of the cross-check in the reference-information display region (120).

11. The sample testing system (10) according to any one of claims 4 to 10, wherein
the controller (51) determines whether or not the visual test is necessary for the sample, basing the result of the analysis on the sample by the sample analyzer (30) and the result of the analysis on the sample by the second sample analyzer (20), and
if determining that the visual test is necessary, the controller (51) displays information indicating that the visual test is necessary for the sample, on the display unit (53).

12. The sample testing system (10) according to claim 11, wherein if determining that the visual test is necessary for the sample, the controller (51) displays information indicating a ground for the determination that the visual test is necessary, in the reference-information display region (120).

13. The sample testing system (10) according to any one of claims 1 to 12, wherein the controller (51) displays a distribution chart indicating distribution of the particle in the sample, in the reference-information display region (120).

14. The sample testing system (10) according to any one of claims 1 to 13, wherein the controller (51) displays results of analyses on the sample in time-series order in the reference-information display region (120).

15. An information processing method of inputting a result of a visual test on a sample containing a particle, comprising the steps of:
displaying a screen including a reference-information display region (120) for a result of an analysis obtained by the sample analyzer (30) of the sample testing system (10) according to any one of the preceding claims, said sample analyzer (30) analyzing the particle in the sample, and an input-value display region (110) for the visual test to be performed on the sample with a microscope, wherein when the controller (51) of the sample testing system (10) has determined that retesting is necessary based on the result of the analysis on the sample then indicating in the reference-information display region (120) that the controller (51) of said sample testing system (10) has determined that retesting is necessary; and
with the screen displayed, receiving a count value of the particle counted in the visual test on the sample and displaying the received count value in the input-value display region (110).

## Patentansprüche

1. Probentestsystem (10), umfassend:
ein Probenanalysegerät (30), das einen Partikel in einer Probe analysiert; und
eine Informationsverarbeitungseinrichtung (50), die ein Ergebnis einer Analyse an der von dem Probenanalysegerät (30) empfangenen Probe empfängt, wobei
die Informationsverarbeitungseinrichtung (50) eine Steuerung (51), eine Anzeigeeinheit (53) und eine Eingabeeinheit (54) umfasst,
das Probentestsystem (10) **dadurch gekennzeichnet ist, dass**:
- die Steuerung (51) die Anzeigeeinheit (53) steuert, um einen Bildschirm anzuzeigen, der einen Referenzinformationsanzeigebereich (120) für das von dem Probenanalysegerät (20, 30) empfangene Ergebnis der Analyse an der Probe und einen Eingabewertanzeigebereich (110) für einen an der Probe mit einem Mikroskop (60) durchzuführenden visuellen Test einschließt, wobei
wenn die Steuerung (51) bestimmt hat, dass eine erneute Testung erforderlich ist, die Steuerung (51) dann die Anzeigeeinheit (53) steuert, um in dem Referenzinformationsanzeigebereich (120) anzuzeigen, dass die Steuerung bestimmt hat, dass eine erneute Testung erforderlich ist, und
- bei angezeigtem Bildschirm, die Steuerung (51) durch die Eingabeeinheit (54) einen Zählwert der beim visuellen Test an der Probe gezählten Partikel empfängt und die Anzeigeeinheit (53) steuert, um den empfangenen Zählwert im Eingabewertanzeigebereich (110) anzuzeigen.

2. Probentestsystem (10) nach Anspruch 1, wobei
das Probenanalysegerät (30) die Probe hinsichtlich Analyseelemente in Bezug auf den Partikel analysiert,
der Referenzinformationsanzeigebereich (120) die Analysewerte der jeweiligen Analyseelemente als Ergebnis der Analyse an der Probe anzeigt,
der Eingabewertanzeigebereich (110) Testelemente und Wertanzeigebereiche für die jeweiligen Testelemente anzeigt, wobei die Testelemente unterklassifizierte Elemente einschließen, die detaillierter sind als die Analyseelemente, und
die Steuerung (51) durch die Eingabeeinheit (54) den Zählwert des beim visuellen Test gezählten Partikels für jedes der Testelemente empfängt und die empfangenen Zählwerte in den Wertanzeigebereichen anzeigt.

3. Probentestsystem (10) nach Anspruch 2, wobei, wenn der Analysewert eines der Analyseelemente für die Probe eine geringe Zuverlässigkeit oder Anomalie darstellt, die Steuerung (51) in Verbindung mit dem Analyseelement Informationen anzeigt, welche die geringe Zuverlässigkeit oder die Anomalie anzeigen.

4. Probentestsystem (10) nach einem der Ansprüche 1 bis 3, weiter umfassend ein zweites Probenanalysegerät (20), das die Probe mit einem anderen Messprinzip als das Probenanalysegerät (30) analysiert, wobei
das Probenanalysegerät (30) die Probe als eine erneute Testung analysiert, nachdem das zweite Probenanalysegerät (20) die Probe analysiert hat, und
die Steuerung (51) weiter ein Ergebnis einer Analyse an der vom zweiten Probenanalysegerät (20,) empfangenen Probe im Referenzinformationsanzeigebereich (120) anzeigt.

5. Probentestsystem (10) nach Anspruch 4, wobei die Steuerung (51) einen ersten Bereich (122) und einen zweiten Bereich (121) nebeneinander innerhalb des Referenzinformationsanzeigebereichs (120) anzeigt, wobei der erste Bereich (122) das Ergebnis der Analyse durch das Probenanalysegerät (30) einschließt und der zweite Bereich (121) das Ergebnis der Analyse durch das zweite Probenanalysegerät (30) einschließt.

6. Probentestsystem (10) nach Anspruch 4 oder 5, wobei
die Steuerung (51) einen ersten Bereich (122) und einen dritten Bereich (123) nebeneinander innerhalb des Referenzinformationsanzeigebereichs anzeigt, wobei der erste Bereich (121, 122) das Ergebnis der Analyse durch das Probenanalysegerät (30) einschließt, der dritte Bereich (123) ein Ergebnis des visuellen Tests an der Probe einschließt, und
wenn die Steuerung einen vorbestimmten Befehl mit dem in dem Eingabewertanzeigebereich (110) angezeigten Zählwert empfängt, die Steuerung den in dem Eingabewertanzeigebereich (110) angezeigten Zählwert als das Ergebnis des visuellen Tests in dem dritten Bereich (123) anzeigt.

7. Probentestsystem (10) nach einem der Ansprüche 4 bis 6, wobei das Probenanalysegerät (30) ein Urinsediment-Analysator (30) und das zweite Probenanalysegerät ein Urinqualität-Analysator (20) ist.

8. Probentestsystem (10) nach Anspruch 7, wobei die Steuerung (51) Analyseelemente in einem ersten Bereich (122) und einem zweiten Bereich (121) innerhalb des Referenzinformationsanzeigebereichs (120) anzeigt, sodass die Analyseelemente in dem ersten Bereich (122) und dem zweiten Bereich (121) in Bezug zueinander horizontal nebeneinander angeordnet sind, wobei der erste Bereich (122) das Ergebnis der Analyse durch den Urinsediment-Analysator (30) einschließt, der zweite Bereich (121) das Ergebnis der Analyse durch den Urinqualität-Analysator (20) einschließt.

9. Probentestsystem (10) nach Anspruch 8, wobei die in Bezug stehenden Analyseelemente im ersten Bereich (122) und im zweiten Bereich (121) eine Kombination sind, die aus einer Gruppe von Kombinationen von okkultem Blut und roten Blutkörperchen, Proteinkonzentration und Abguss, Nitrit und Bakterium sowie spezifischem Gewicht und elektrischer Leitfähigkeit ausgewählt ist.

10. Probentestsystem (10) nach einem der Ansprüche 7 bis 9, wobei die Steuerung (51) einen Kreuzvergleich zwischen dem Ergebnis der Analyse durch den Urinqualität-Analysator (20) und dem Ergebnis der Analyse durch den Urinsediment-Analysator (30) durchführt und ein Ergebnis des Kreuzvergleichs in dem Referenzinformationsanzeigebereich (120) anzeigt.

11. Probentestsystem (10) nach einem der Ansprüche 4 bis 10, wobei
die Steuerung (51) basierend auf dem Ergebnis der Analyse an der Probe durch das Probenanalysegerät (30) und dem Ergebnis der Analyse an der Probe durch das zweite Probenanalysegerät (20) bestimmt, ob der visuelle Test für die Probe erforderlich ist oder nicht, und
wenn bestimmt wird, dass der visuelle Test erforderlich ist, die Steuerung (51) auf der Anzeigeeinheit (53) Informationen anzeigt, die angeben, dass der visuelle Test für die Probe erforderlich ist.

12. Probentestsystem (10) nach Anspruch 11, wobei, wenn bestimmt wird, dass der visuelle Test für die Probe erforderlich ist, die Steuerung (51) im Referenzinformationsanzeigebereich (120) Informationen anzeigt, die einen Grund für die Bestimmung anzeigen, dass der visuelle Test erforderlich ist.

13. Probentestsystem (10) nach einem der Ansprüche 1 bis 12, wobei die Steuerung (51) in dem Referenzinformationsanzeigebereich (120) ein Verteilungsdiagramm anzeigt, das die Verteilung des Partikels in der Probe angibt.

14. Probentestsystem (10) nach einem der Ansprüche 1 bis 13, wobei die Steuerung (51) die Ergebnisse der Analysen an der Probe in zeitlicher Abfolge im Referenzinformationsanzeigebereich (120) anzeigt.

15. Informationsverarbeitungsverfahren zum Eingeben eines Ergebnisses eines visuellen Tests an einer Probe, die ein Partikel enthält, umfassend die folgenden Schritte:
Anzeigen eines Bildschirms, der einen Referenzinformationsanzeigebereich (120) für ein Ergebnis einer Analyse einschließt, die durch das Probenanalysegerät (30) des Probentestsystems (10) nach einem der vorstehenden Ansprüche erhalten wird, wobei das Probenanalysegerät (30) den Partikel in der Probe analysiert, und einen Eingabewertanzeigebereich (110) für den an der Probe mit einem Mikroskop durchzuführenden visuellen Test, wobei, wenn die Steuerung (51) des Probentestsystems (10) basierend auf dem Ergebnis der Analyse an der Probe bestimmt hat, dass eine erneute Testung erforderlich ist, im Referenzinformationsanzeigebereich (120) dann angezeigt wird, dass die Steuerung (51) des Probentestsystems (10) bestimmt hat, dass eine erneute Testung erforderlich ist; und
bei angezeigtem Bildschirm, Empfangen eines Zählwertes der im visuellen Test an der Probe gezählten Partikel und Anzeigen des empfangenen Zählwertes im Eingabewertanzeigebereich (110).

## Revendications

1. Système de test d'échantillon (10) comprenant :
un analyseur d'échantillon (30) qui analyse une particule dans un échantillon ; et
un appareil de traitement d'informations (50) qui reçoit un résultat d'une analyse sur l'échantillon reçu à partir de l'analyseur d'échantillon (30), dans lequel
l'appareil de traitement d'informations (50) comprend un dispositif de commande (51), une unité d'affichage (53), et une unité d'entrée (54),
le système de test d'échantillon (10) étant **caractérisé en ce que** :
- le dispositif de commande (51) commande l'unité d'affichage (53) pour afficher un écran incluant une région d'affichage d'informations de référence (120) pour le résultat de l'analyse sur l'échantillon, reçu à partir de l'analyseur d'échantillon (20, 30), et une région d'affichage de valeur d'entrée (110) pour un test visuel à effectuer sur l'échantillon avec un microscope (60), dans lequel
quand le dispositif de commande (51) a déterminé qu'un nouveau test est nécessaire, alors le dispositif de commande (51) commande l'unité d'affichage (53) pour indiquer dans la région d'affichage d'informations de référence (120) que le dispositif de commande a déterminé qu'un nouveau test est nécessaire, et
- avec l'écran affiché, le dispositif de commande (51) reçoit une valeur de comptage de la particule compté dans le test visuel sur l'échantillon, par l'intermédiaire de l'unité d'entrée (54), et commande l'unité d'affichage (53) pour afficher la valeur de comptage reçue dans la région d'affichage de valeur d'entrée (110).

2. Système de test d'échantillon (10) selon la revendication 1, dans lequel
l'analyseur d'échantillon (30) analyse l'échantillon en termes d'éléments d'analyse associés à la particule,
la région d'affichage d'informations de référence (120) affiche des valeurs d'analyse des éléments d'analyse respectifs en tant que résultat de l'analyse sur l'échantillon,
la région d'affichage de valeur d'entrée (110) affiche des éléments de test et des régions d'affichage de valeur pour les éléments de test respectifs, les éléments de test incluant des éléments sous-classés qui sont plus détaillés que les éléments d'analyse, et
le dispositif de commande (51) reçoit, par l'intermédiaire de l'unité d'entrée (54), la valeur de comptage de la particule comptée dans le test visuel pour chacun des éléments de test et affiche les valeurs de comptage reçues dans les régions d'affichage de valeur.

3. Système de test d'échantillon (10) selon la revendication 2, dans lequel quand la valeur d'analyse de l'un quelconque des éléments d'analyse pour l'échantillon représente une faible fiabilité ou une anomalie, le dispositif de commande (51) affiche des informations indiquant la faible fiabilité ou l'anomalie, en association avec l'élément d'analyse.

4. Système de test d'échantillon (10) selon l'une quelconque des revendications 1 à 3, comprenant en outre un second analyseur d'échantillon (20) qui analyse l'échantillon avec un principe de mesure différent de celui de l'analyseur d'échantillon (30), dans lequel
l'analyseur d'échantillon (30) analyse l'échantillon dans le cadre d'un nouveau test après que le second analyseur d'échantillon (20) a analysé l'échantillon, et
le dispositif de commande (51) affiche en outre un résultat d'une analyse sur l'échantillon reçu à partir du second analyseur d'échantillon (20), dans la région d'affichage d'informations de référence (120).

5. Système de test d'échantillon (10) selon la revendication 4, dans lequel le dispositif de commande (51) affiche une première région (122) et une deuxième région (121) l'une à côté de l'autre dans la région d'affichage d'informations de référence (120), la première région (122) incluant le résultat de l'analyse par l'analyseur d'échantillon (30), la deuxième région (121) incluant le résultat de l'analyse par le second analyseur d'échantillon (30).

6. Système de test d'échantillon (10) selon la revendication 4 ou 5, dans lequel
le dispositif de commande (51) affiche une première région (122) et une troisième région (123) l'une à côté de l'autre dans la région d'affichage d'informations de référence, la première région (121, 122) incluant le résultat de l'analyse par l'analyseur d'échantillon (30), la troisième région (123) incluant un résultat du test visuel sur l'échantillon, et
lors de la réception d'une commande prédéterminée avec la valeur de comptage affichée dans la région d'affichage de valeur d'entrée (110), le dispositif de commande affiche la valeur de comptage affichée dans la région d'affichage de valeur d'entrée (110), en tant que résultat du test visuel dans la troisième région (123).

7. Système de test d'échantillon (10) selon l'une quelconque des revendications 4 à 6, dans lequel l'analyseur d'échantillon (30) est un analyseur de sédiment urinaire (30), et le second analyseur d'échantillon est un analyseur qualitatif d'urine (20).

8. Système de test d'échantillon (10) selon la revendication 7, dans lequel le dispositif de commande (51) affiche des éléments d'analyse dans une première région (122) et une deuxième région (121) dans la région d'affichage d'informations de référence (120) de sorte que les éléments d'analyse dans la première région (122) et la deuxième région (121) associés les uns aux autres sont agencés les uns à côté des autres horizontalement, la première région (122) incluant le résultat de l'analyse par l'analyseur de sédiment urinaire (30), la deuxième région (121) incluant le résultat de l'analyse par l'analyseur qualitatif d'urine (20).

9. Système de test d'échantillon (10) selon la revendication 8, dans lequel les éléments d'analyse associés dans la première région (122) et la deuxième région (121) sont une combinaison sélectionnée dans un groupe de combinaisons de sang occulte et de globules rouges, de concentration en protéines et de coulée, de nitrite et de bactérie, et de densité spécifique et de conductivité électrique.

10. Système de test d'échantillon (10) selon l'une quelconque des revendications 7 à 9, dans lequel le dispositif de commande (51) effectue une contre-vérification entre le résultat de l'analyse par l'analyseur qualitatif d'urine (20) et le résultat de l'analyse par l'analyseur de sédiment urinaire (30), et affiche un résultat de la contre-vérification dans la zone d'affichage d'informations de référence (120).

11. Système de test d'échantillon (10) selon l'une quelconque des revendications 4 à 10, dans lequel
le dispositif de commande (51) détermine si le test visuel est nécessaire ou non pour l'échantillon, en se basant sur le résultat de l'analyse sur l'échantillon par l'analyseur d'échantillon (30) et le résultat de l'analyse sur l'échantillon par le second analyseur d'échantillon (20), et
s'il est déterminé que le test visuel est nécessaire, le dispositif de commande (51) affiche des informations indiquant que le test visuel est nécessaire pour l'échantillon, sur l'unité d'affichage (53).

12. Système de test d'échantillon (10) selon la revendication 11, dans lequel s'il est déterminé que le test visuel est nécessaire pour l'échantillon, le dispositif de commande (51) affiche des informations indiquant une raison de la détermination que le test visuel est nécessaire, dans la région d'affichage d'informations de référence (120).

13. Système de test d'échantillon (10) selon l'une quelconque des revendications 1 à 12, dans lequel le dispositif de commande (51) affiche un diagramme de distribution indiquant la distribution de la particule dans l'échantillon, dans la région d'affichage d'informations de référence (120).

14. Système de test d'échantillon (10) selon l'une quelconque des revendications 1 à 13, dans lequel le dispositif de commande (51) affiche les résultats d'analyses sur l'échantillon dans un ordre chronologique dans la région d'affichage d'informations de référence (120).

15. Procédé de traitement d'informations consistant à entrer un résultat d'un test visuel sur un échantillon contenant une particule, comprenant les étapes consistant à :
afficher un écran incluant une région d'affichage d'informations de référence (120) pour un résultat d'une analyse obtenue par l'analyseur d'échantillon (30) du système de test d'échantillon (10) selon l'une quelconque des revendications précédentes, ledit analyseur d'échantillon (30) analysant la particule dans l'échantillon, et une région d'affichage de valeur d'entrée (110) pour le test visuel à effectuer sur l'échantillon avec un microscope, dans lequel quand le dispositif de commande (51) du système de test d'échantillon (10) a déterminé qu'un nouveau test est nécessaire sur la base du résultat de l'analyse sur l'échantillon, alors indiquer dans la région d'affichage d'informations de référence (120) que le dispositif de commande (51) dudit système de test d'échantillon (10) a déterminé qu'un nouveau test est nécessaire ; et
avec l'écran affiché, recevoir une valeur de comptage de la particule comptée dans le test visuel sur l'échantillon et afficher la valeur de comptage reçue dans la région d'affichage de valeur d'entrée (110).
